# DEMANDE DE BREVET EUROPEEN

(11) **EP 0 691 339 A1**
(43) Date de publication de la demande: **10.01.1996**
(21) Numéro de dépôt: 95401608.5
(22) Date de dépôt: 05.07.1995
(51) Int. Cl.: C07D 471/04, C07D 498/04, A61K 31/44

(54) **Dérivés amines de 1,3-dihydro-2H-pyrrolo(2,3-b)pyridin-2-ones et oxazolo(4,5-b)pyridin-2(3H)-ones, leur procédé de préparation et les compositions pharmaceutiques qui les contiennent**

(30) Priorité: 07.07.1994 FR 9408419; 07.07.1994 FR 9408418
(71) Demandeur: ADIR ET COMPAGNIE, F-92415 Courbevoie Cédex (FR)
(72) Inventeur: Guillaumet, Gérald, F-46450 Saint Jean le Blanc (FR); Viaud, Marie-Claude, F-45000 Orleans (FR); Savelon, Laurence, F-45800 Saint Jean de Braye (FR); Pavli, Panayota, GR-117-41 Coukaki Athenes (GR); Renard, Pierre, F-78000 Versailles (FR); Pfeiffer, Bruno, F-95600 Eaubonne (FR); Caignard, Daniel-Henri, F-75015 Paris (FR); Bizot-Espiard, Jean-Guy, F-75015 Paris (FR); Adam, Gérard, F-78600 le Mesnil le Roi (FR)

(57) **Abrégé**

L'invention concerne les composés de formule générale (I) : 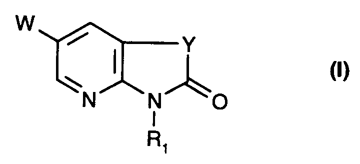
dans laquelle R₁, W, et Y sont tels que définis dans la description, leurs isomères géométriques et/ou optiques, et leurs sels d'addition à un acide ou une base, pharmaceutiquement acceptables.

Médicaments.

## Description

La présente invention concerne de nouveaux dérivés de 1,3-dihydro-*2H*-pyrrolo[2,3-b]pyridin-2-ones et oxazolo[4,5-b]pyridin-*2(3H)*-ones, leur procédé de préparation et les compositions pharmaceutiques qui les contiennent.

De nombreux dérivés des 1,3-dihydro-*2H*-pyrrolo[2,3-b]pyridin-2-ones et oxazolo[4,5-b]pyridin-*2(3H)*-ones sont décrits en thérapeutique comme possédant des activités telles que cardiotoniques, insecticides ou anti-inflammatoires.
• Pour ce qui est des 1,3-dihydro-*2H*-pyrrolo[2,3-b]pyridin-2-ones, le brevet EP-436333 revendique notammant des 3-aminocarbonyl-1,3-dihydro-*2H*-pyrrolo[2,3-b]pyridin-2-ones décrites comme possédant d'intéressantes propriétés anti-inflammatoires.

Des 1,3-dihydro-*2H*-pyrrolo[2,3-b]pyridin-2-ones substituées en position 3 par des alkyles et des aryles sont également décrites comme anti-inflammatoires dans un article du Journal of Medicinal Chemistry, (1990), *33*, 2697-2706.
• Pour ce qui est des oxazolo[4,5-b]pyridin-*2(3H)*-ones, et plus particulièrement des composés substitués au niveau du noyau pyridine, les brevets EP-357 675 et US-486 674 revendiquent un certain nombre de composés substitués en position 6 par des pyridines et décrits comme étant cardiotoniques.

Les composés de la présente invention présentent une haute originalité structurale de par les groupements acylés, hydroxylés et/ou aminés qui les substituent sur la partie pyridinique de l'hétérocycle.

Aucune oxazolo[4,5-b]pyridin-*2(3H)*-one de ce type n'est décrite ou revendiquée dans la littérature, et les seules pyrrolo[2,3-b]pyridin-2-ones substituées sur le noyau pyridine ne le sont que par des halogènes, alkyles ou nitriles et sont décrites comme étant essentiellement anti-inflammatoires.

Les nouveaux composés découverts par la demanderesse se différencient de la totalité des dérivés évoqués précédemment par leurs puissantes propriétés antalgiques associées à une quasi absence de propriétés anti-inflammatoires.

La plupart des substances analgésiques non morphiniques connues à ce jour possèdent également une activité antiinflammatoire et interviennent donc sur les processus liés aux phénomènes d'inflammation (c'est le cas par exemple des dérivés salicylés comme l'aspirine, les pyrazolés comme la phénylbutazone, les acides arylacétiques ou hétéroarylacétiques comme l'indométhacine...). Etant anti-inflammatoires, ces substances inhibent la cyclooxygénase ce qui provoque un bloquage de la biosynthèse de nombreux médiateurs chimiques (prostaglandines, prostacycline, thromboxane A2...). Il s'ensuit donc de multiples effets secondaires, parmi lesquels l'inhibition de l'agrégation plaquettaire associée à des troubles de la coagulation et une toxicité gastro intestinale avec possibilité d'ulcérations et d'hémorragie due à une diminution de la biosynthèse des prostaglandines PGE₂ et PGF_{1α} qui sont cytoprotectrices de la muqueuse gastrique. Outre les désagréments qu'ils occasionnent, ces effets secondaires peuvent, chez de nombreux sujets qui y sont particulièrement sensibles, rendre impossible la prescription de substances antalgiques dotées de propriétés anti-inflammatoires.

Les composés de la présente invention n'intervenant pas sur les médiateurs de l'inflammation sont donc dépourvus des effets secondaires mentionnés précédemment.

Cette caractéristique associée à leur absence de toxicité et à leur haut niveau d'activité rend les composés de la présente invention utilisables en tant qu'analgésiques sans les restrictions d'usage habituellement en vigueur pour la majorité des produits de cette classe.

Plus spécifiquement l'invention concerne les composés de formule générale (I) : 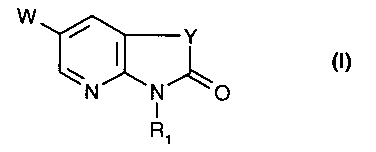
dans laquelle :
- R₁ est choisi parmi l'hydrogène un radical alkyle, alcényle, cyanoalkyle et arylalkyle,
- W est choisi parmi les groupements ―A―R₂ et 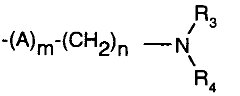
- R₂ est choisi parmi un groupement alkyle, cycloalkyle, cycloalkylalkyle, phényle, phénylalkyle, naphtyle et naphtylalkyle,
- R₃ et R₄ sont choisis indépendamment l'un de l'autre parmi l'hydrogène, un groupement alkyle, phényle, phénylalkyle, cycloalkyle et cycloalkylalkyle ou forment ensemble avec l'atome d'azote qui les porte un système hétérocyclique choisi parmi : 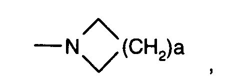 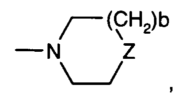 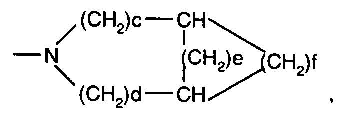 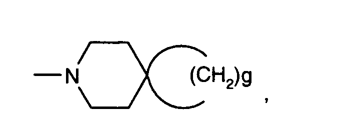 dans lesquels :
   - a représente un entier compris entre 0 et 4,
   - b représente 1 ou 2,
   - c, d, e, f, représentent des nombres entiers compris entre 0 et 4,
   - g représente 4 ou 5,
   - Z représente O, S ou N-R₇ dans lequel R₇ représente un atome d'hydrogène, un groupement alkyle, phényle, phénylalkyle, cycloalkyle, cycloalkylalkyle, benzhydryle, naphtyle, pyridyle, pyrimidyle,
- n représente un entier de 1 à 4 inclus,
- m représente 0 ou 1,
- A est choisi parmi le groupement 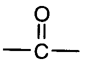 et le groupement 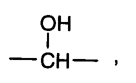
- Y représente un atome d'oxygène ou un groupe 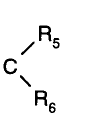 dans lequel R₅ et R₆ sont choisis indépendemment l'un de l'autre parmi l'hydrogène, un radical alkyle, cycloalkyle, cycloalkylalkyle, le radical phényle et le radical benzyle,

étant entendu que lors de la description de la formule (I) :
- les termes "alkyle", "alcényle" et "alkoxy" désignent des groupements linéaires ou ramifiés contenant de 1 à 6 atomes de carbone et pouvant être non substitués ou substitués par un ou plusieurs radicaux alkoxy,
- le terme "aryle" désigne les radicaux phényle, naphtyle ou pyridine,
- les radicaux phényle, benzyle, phénylalkyle, naphtyle, pyridine, pyrimidyle et benzhydryle peuvent être non substitués ou substitués par un ou plusieurs atomes d'halogène ou radicaux hydroxyle, alkyle, alkoxy, trifluorométhyle ou nitro,
- le terme "cycloalkyle" désigne un système cyclique comportant de 3 à 8 atomes de carbone,
- les termes "cycloalkylalkyle", "arylalkyle", "phénylalkyle" et "naphtylalkyle" désignent un cycloalkyle, un aryle, un phényle ou un naphtyle rattaché par l'intermédiaire d'une chaine carbonée linéaire ou ramifiée comportant de 1 à 6 atomes de carbone,
- le système hétérocyclique formé par R₃ et R₄ peut être non substitué ou substitué par un ou plusieurs atomes d'halogène ou radicaux alkyle,

leurs éventuels isomères géométriques et/ou optiques, sous forme pure ou sous forme de mélange, et leurs sels d'addition à un acide ou une base, pharmaceutiquement acceptables.

La présente invention concerne également le procédé de préparation des composés de formule (I) caractérisé en ce que l'on fait réagir :
***A. lorsque W représente -A-R₂,*** un composé de formule (II) : 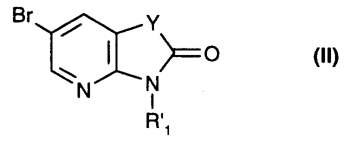 dans laquelle Y est tel que défini dans la formule (I) et R'₁ a la même définition que R₁ avec la réserve que R'₁ ne peut représenter un atome d'hydrogène,
   - soit en présence de 1,2-bis(diphénylphosphino)éthane et d'acétate de palladium (II) avec un éther insaturé de formule (III) :

      Alk―O―CH=CH―R'₂ **(III)**

      dans laquelle Alk représente un alkyle de 1 à 4 atomes de carbone et R'₂ représente un hydrogène ou un alkyle linéaire ou ramifié de 1 à 5 atomes de carbone éventuellement substitué par un phényle, naphtyle ou cycloalkyle de manière à obtenir les composés de formule (IV) : 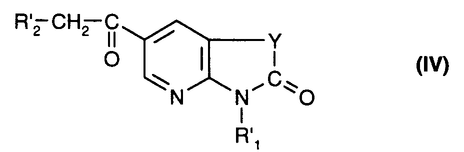 dans laquelle R'₁, R'₂ et Y sont tels que définis précédemment,
   - soit en présence de tétrakis(triphénylphosphine)palladium et de chlorure de lithium avec un composé de formule (V) : 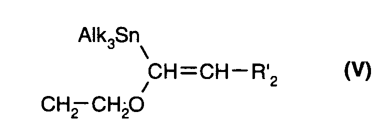 dans laquelle R'₂ et Alk ont la même définition que précédemment de manière à accéder également aux composés de formule (IV) : 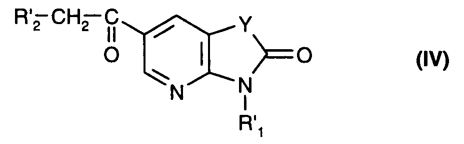
   - soit en présence de zinc avec un dérivé halogéné de formule (VI) :

      Ar-CH₂―Hal **(VI)**

      dans laquelle Hal représente un atome d'halogène et Ar un groupement phényle ou naphtyle éventuellement substitué de manière à obtenir les composés de formule (VII) : 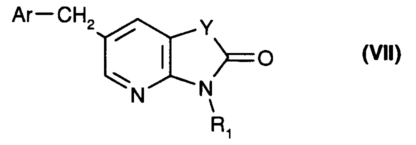 dans laquelle Ar est tel que défini précédemment et R₁ est tel que défini dans la formule (I),
   que l'on soumet à une réaction d'oxydation avec un agent oxydant comme par exemple l'oxyde de chrome ou le N-bromosuccinimide pour accéder aux composés de formule (VIII) : 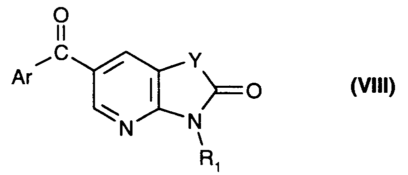 dans laquelle Ar, Y et R₁ sont tels que défini précédemment,
   avec dans le cas où le N-bromosuccinimide est utilisé et où Y représente le groupement CH₂, nécessité de faire suivre la réaction d'oxydation d'une étape de débromation avec du zinc,
   les composés de formules (IV) et (VIII) pouvant si on le désire :
   - dans le cas où R₁ ou R'₁ représentent un groupement benzyle, être débenzylés en présence de palladium sur charbon et hydrogène pour accéder aux composés de formules (IX) et (X) : 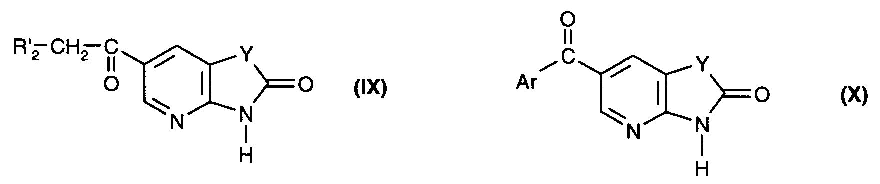 dans lesquelles Y, Ar et R'₂ sont tels que définis précédemment,
   - dans le cas où R₁ ou R'₁ représentent un groupement cyanométhylé, être décyanométhylés en présence d'oxyde de platine et d'hydrogène pour accéder à ces mêmes composés de formules (IX) et (X),
   l'ensemble de ces composés (IV), (VIII), (IX), (X) constituant les composés de formule (XI) : 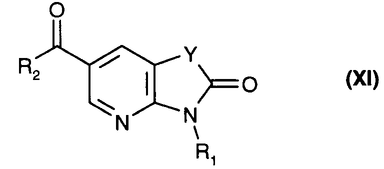 dans laquelle R₁, R₂ et Y sont tels que définis précédemment,
   composés de formule (XI) qui peuvent, si on le désire, être réduits par un agent réducteur, comme le borohydrure de sodium, en alcool de formule (XII) : 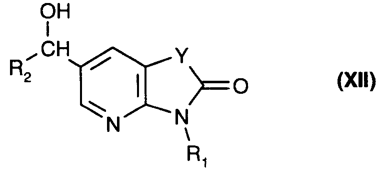 dans laquelle R₁, R₂ et Y sont tels que définis précédemment,
***B. lorsque W représente*** 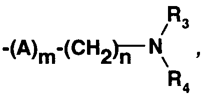 un composé de formule (XIII) : 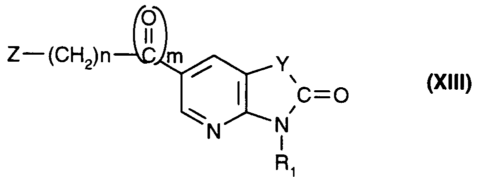 dans laquelle Y, R₁, n et m sont tels que définis pour la formule (I) et Z représente un groupement partant tel qu'un atome d'halogène ou un tosylate, avec une amine de formule (XIV) : 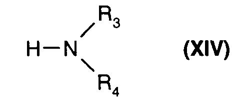 dans laquelle R₃ et R₄ sont tels que définis pour la formule (I),
   pour obtenir un composé de formule (XV) : 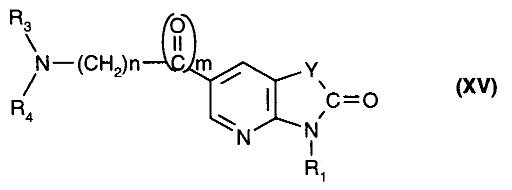 dans laquelle n, m, Y, R₁, R₃ et R₄ sont tels que défini précédemment,
   dont on peut éventuellement, dans le cas où m = 1, réduire la fonction carbonyle avec un agent réducteur comme par exemple du borohydrure de sodium pour obtenir l'alcool correspondant de formule (XVI) : 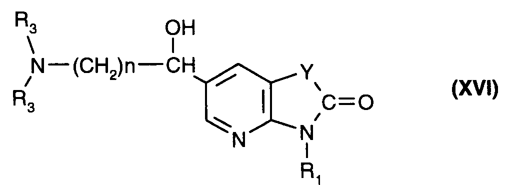 dans laquelle n, Y, R₁, R₂ et R₃ sont tels que définis précédemment,
   l'ensemble des composés de formules (XI), (XII), (XV) et (XVI) formant les composés de formule (I) que l'on purifie le cas échéant par une technique classique de purification, dont on sépare, si on le souhaite, les isomères géométriques et les isomères optiques par une technique classique de séparation et que l'on transforme, si nécessaire, en leurs sels d'addition à un acide ou à une base, pharmaceutiquement acceptables.

L'étude pharmacologique des composés de l'invention a montré qu'ils sont peu toxiques, doués d'une haute activité analgésique pure et donc dépourvus des inconvénients inhérents à la composante anti-inflammatoire des composés non morphiniques présentant ce type d'activité (action ulcérigène, perturbation des processus de coagulation...).

Cette activité analgésique pure rend les composés de la présente invention très intéressants dans nombres d'indications telles que : algies rhumatismales, névralgies lombosciatiques, névralgies cervico-brachiales, algies traumatiques telles que entorses, fractures, luxations, douleurs post-traumatiques, douleurs post-opératoires, douleurs dentaires, douleurs neurologiques telles que névralgie faciales, douleurs viscérales telles que coliques néphrétiques, dysménorrhées, chirurgie proctologique, douleurs de la sphère O.R.L., pancréatites, algies diverses, céphalées, douleurs des cancéreux...

La présente invention a également pour objet les compositions pharmaceutiques contenant l'un des composés de formule (I) sous forme de base ou salifié par un acide pharmaceutiquement acceptable, seul ou en combinaison avec un ou plusieurs excipients ou véhicules inertes non toxiques, pharmaceutiquement acceptables.

Sont également compris dans l'invention les compositions pharmaceutiques contenant l'un des composés de formule (I), sous forme de base ou salifié, associé à de la caféine et en combinaison avec un ou plusieurs excipients ou véhicules inertes non toxiques pharmaceutiquement acceptables.

Parmi les compositions pharmaceutiques selon l'invention, on pourra citer plus particulièrement celles qui conviennent pour l'administration orale, parentérale, nasale, rectale, perlinguale, oculaire ou respiratoire et notamment les préparations injectables, les aérosols, les gouttes occulaires ou nasales, les comprimés simples ou dragéifiés, les comprimés sublinguaux, les sachets, les paquets, les gélules, les glossettes, les tablettes, les suppositoires, les crèmes, les pommades, gels dermiques etc...

La posologie utile varie selon l'âge et le poids du patient, la voie d'administration, la nature de l'indication thérapeutique et des traitements éventuellement associés et se situe entre 5 mg et 4 g par 24 heures.

Les exemples suivants illustrent l'invention et ne la limitent en aucune manière.

### PREPARATION 1 : 6-BROMO-OXAZOLO[4,5-b]PYRIDIN-2(3H)-ONE

Après dissolution de l'oxazolo[4,5-b]pyridin-*2(3H)*-one (5 g ; 23,25 mmol) dans le N,N-diméthylformamide (100 ml), le brome (1,28 ml ; 25,58 mmol) est ajouté lentement. L'agitation est maintenue à température ambiante pendant 2 heures. Puis on ajoute de l'eau (50 ml) au milieu réactionnel ; le produit du titre est isolé après avoir été filtré, rincé avec un peu d'eau et séché sous vide. Le rendement obtenu est de 90 %. 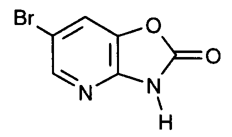
F : 229-230°C
IR (KBr) : 1750 cm⁻¹ (CO carbamate)
RMN ¹H (CDCl₃+D₂O), δ (ppm) : 7,54 (d, H₇, 1H, J_{5,7}=2,4 Hz) ; 8,17 (d, H₅, 1H, J_{5,7}=2,4 Hz)

### PREPARATION 2 : 6-BROMO-3-METHYLOXAZOLO[4,5-b]PYRIDIN-2(3H)-ONE

La 6-bromo-oxazolo[4,5-b]pyridin-*2(3H)*-one de la préparation 1 (530 mg ; 2 mmol) est dissoute dans le N,N-diméthylformamide (10 ml), on ajoute alors l'hydrure de sodium (80 % dans l'huile) (66 mg ; 2,20 mmol). L'agitation est maintenue à température ambiante pendant 1 heure puis l'iodure de méthyle (426 mg ; 3 mmol) dilué avec du N,N-diméthylformamide (0,5 ml) est additionné goutte-à-goutte. Le mélange réactionnel est porté à reflux pendant 2 heures. Lorsque la solution est refroidie et le solvant évaporé sous pression réduite, le résidu est repris avec de l'eau et extrait avec du dichlorométhane. La phase organique est séchée sur sulfate de magnésium, filtrée, puis le solvant est évaporé. Le produit du titre est purifié par chromatographie sur gel de silice (éluant : dichlorométhane). Le rendement obtenu est de 85 %. 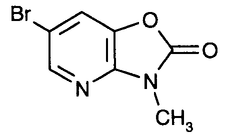
F : 125-127°C
IR (KBr) : 1770 cm⁻¹ (CO carbamate)
RMN ¹H (CDCl₃), δ (ppm) : 3,47 (s, NCH₃, 3H) ; 7,52 (d, H₇, 1H, J_{5,7}=2,2 Hz) ; 8,16 (d, H₅, 1H, J_{5,7}=2,2 Hz).

### PREPARATION 3 : 6-BROMO-3-(CYANOMETHYL)OXAZOLO[4,5-b]PYRIDIN-2(3H)-ONE

La 6-bromo-oxazolo[4,5-b]pyridin-*2(3H)*-one de la préparation 1 (215 mg ; 1,0 mmol) est additionnée à une solution d'éthanolate de sodium préparée avec du sodium (28 mg ; 1,2 mmol) dans de l'éthanol anhydre (6 ml). Le mélange réactionnel est agité à température ambiante pendant 1 heure, puis l'éthanol est évaporé sous pression réduite. L'anion est alors dissous dans le N,N-diméthylformamide (6 ml) puis le bromoacétonitrile (0,1 ml ; 1,5 mmol) dilué avec un peu de solvant est ajouté goutte-à-goutte. Le mélange réactionnel est porté à reflux pendant 2 heures. Lorsque la solution est refroidie et le solvant évaporé sous pression réduite, le résidu est repris avec de l'eau et extrait avec du dichlorométhane. La phase organique est séchée sur sulfate de magnésium, filtrée puis le solvant est évaporé. Le produit du titre est purifié par chromatographie sur gel de silice (éluant : dichlorométhane). Le rendement obtenu est de 82 %. 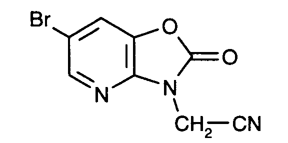
F : 127-129°C
IR (KBr) : 1780 cm⁻¹ (CO carbamate),
RMN ¹H (CDCl₃), δ (ppm) : 4,81 (s, NCH₂, 2H) ; 7,66 (d, H₇, 1H, J_{5,7}=1,5 Hz) ; 8,28 (d, H₅, 1H, J_{5,7}=1,5 Hz)

### PREPARATION 4 : 2-(BENZYLOXY)-6-BROMOOXAZOLO[4,5-b]PYRIDINE

Le mode opératoire est le même que celui utilisé lors de la synthèse du composé de la préparation 2. Le bromure de benzyle est employé à la place de l'iodure de méthyle. Le rendement obtenu est de 60 %. 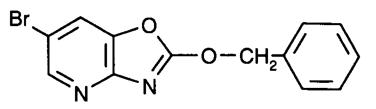
F : 222-224°C
IR (KBr) : 1740 cm⁻¹ (-C=N-)
RMN ¹H (CDCl₃), δ (ppm) : 5,40 (s, NCH₂, 2H) ; 7,16 (d, H₇, 1H, J_{5,7}=1,5 Hz) ;
7,34 (s, Hₐᵣₒₘ, 5H) ; 7,42 (d, H₅, 1H, J_{5,7}=1,5 Hz).
MS (IC/NH₃) : m/z=307 (M+1)

### PREPARATION 5 : 6-BROMO-3-(2-PHENYLETHYL)OXAZOLO[4,5-b]PYRIDIN-2(3H)-ONE

- **Méthode A :**
   En procédant de la même façon que pour la synthèse du composé de la préparation 2, mais en remplaçant l'iodure de méthyle par le (2-bromoéthyl)benzène, on obtient le composé du titre avec un rendement de 63 %.
- **Méthode B :**
   En procédant de la même façon que pour la synthèse du composé de la préparation 3, mais en remplaçant le bromoacétonitrile par le (2-bromoéthyl)benzène, on obtient le composé du titre avec un rendement de 78 %.

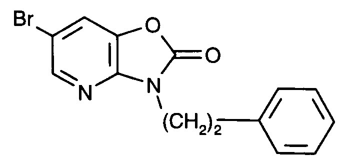

F : 115-117°C
IR (KBr) : 1780 cm⁻¹ (CO carbamate)
RMN ¹H (CDCl₃), δ (ppm) : 3,14 (dd, CH₂, 2H, J₁=8,1 Hz, J₂=7,4 Hz) ;
4,15 (dd, NCH₂, 2H, J₁ = 8,1 Hz J₂=7,4 Hz) ; 7,18-7,34 (m, Hₐᵣₒₘ, 5H) ;
7,55 (d, H₇, 1H, J_{5,7}=2,2 Hz) ; 8,19 (d, H₅, 1H, J_{5,7}=2,2 Hz)

### PREPARATION 6 : 3-ALLYL-6-BROMO-OXAZOLO[4,5-b]PYRIDIN-2(3H)-ONE

- **Méthode A :**
   En procédant de la même façon que pour la synthèse du composé de la préparation 2, mais en remplaçant l'iodure de méthyle par le bromure d'allyle, on obtient le composé du titre avec un rendement de 41 %.
- **Méthode B :**
   En procédant de la même façon que pour la synthèse du composé de la préparation 3, mais en remplaçant le bromoacétonitrile par le bromure d'allyle, on obtient le composé du titre avec un rendement de 56%.

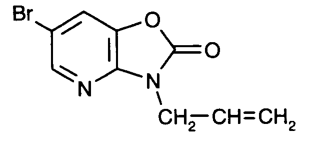

F : 71-73°C
IR (KBr) : 1785 cm⁻¹ (CO carbamate)
RMN ¹H (CDCl₃), δ (ppm) : 4,52 (d NCH₂, 2H, J=5,9 Hz) ; 5,29 (d, H_{éthyl.}, 1H, J=9,9 Hz) ;
5,33 (d, H_{éthyl.}, 1H, J=17,5 Hz) ; 5,89-6,04 (m, H_{éthyl.}, 1H) ;
7,57 (d, H₇, 1H, J_{5,7}=2,2 Hz) ; 8,20 (d, H₅, 1H, J_{5,7}=1,5 Hz)

### PREPARATION 7 : 6-BROMO-3-(2-CYANOETHYL)OXAZOLO[4,5-b]PYRIDIN-2(3H)-ONE

La 6-bromo-oxazolo[4,5-b]pyridin-*2(3H)*-one (215 mg ; 1mmol) est dissoute dans le N,N-diméthylformamide (15 ml). L'acrylonitrile (64 mg ; 1,2 mmol) ainsi que la triéthylamine (120 mg ; 1,2 mmol) sont successivement ajoutés à la solution. Le milieu réactionnel est maintenu à reflux pendant 12 heures. Lorsque la solution est refroidie et le solvant évaporé sous pression réduite, le résidu est repris avec de l'eau et extrait avec du dichlorométhane. La phase organique est séchée sur sulfate de magnésium, filtrée puis le solvant est évaporé. Le produit du titre est purifié par chromatographie sur gel de silice (éluant : chlrorure de méthylène). Le rendement obtenu est de 81 %. 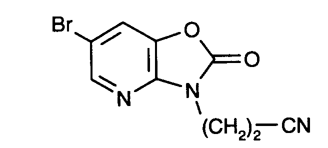
F : 146-148°C
IR (KBr) : 2240 cm⁻¹ (CN), 1785 cm⁻¹ (CO carbamate)
RMN ¹H (CDCl₃),δ (ppm) : 2,97 (dd, CH₂, 2H, J₁=7,4 Hz, J₂=6,6 Hz) ;
4,24 (dd, NCH₂, 2H, J₁=7,4 Hz J₂=6,6 Hz) ; 7,61 (d, H₇, 1H, J_{5,7}=1,5 Hz) ;
8,21 (d, H₅, 1H, J_{5,7}=1,5 Hz)

### PREPARATION 8 : 6-BROMO-3-[2-(PYRIDIN-2-YL)ETHYL]OXAZOLO[4,5-b]PYRIDIN-2(3H)-ONE

La 6-bromo-oxazolo[4,5-b]pyridin-*2(3H)*-one (1 g ; 4,65 mmol) est mise en suspension dans la 2-vinylpyridine (5 ml). La solution est chauffée, sous forte agitation, jusqu'à la dissolution complète du dérivé bromé. Le chauffage à reflux est maintenu pendant 2 heures. Lorsque la solution est refroidie, reprendre avec de l'eau et extraire avec du dichlorométhane. La phase organique est séchée sur sulfate de magnésium, filtrée puis le solvant est évaporé. Le produit du titre est purifié par chromatographie sur gel de silice (éluant : dichlorométhane/acétate d'éthyle : 9/1). Le rendement obtenu est de 87 %. 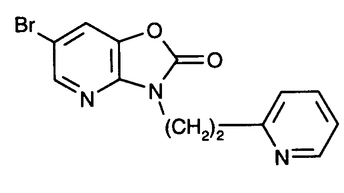
F : 130-132°C
IR (KBr) : 1780 cm⁻¹ (CO carbamate)
RMN ¹H (CDCl₃), δ (ppm) : 3,31 (t, CH₂, 2H, J=7,4 Hz) ; 4,34 (t, NCH₂, 2H, J=7,4 Hz) ;
7,08-7,16 (m, Hₐᵣₒₘ, 2H) ; 7,52 (d, H₇, 1H, J_{5,7}=1,5 Hz) ; 7,52-7,60 (t, Hₐᵣₒₘ, 1H, J=7,4 Hz) ; 8,15 (d, H₅, 1H, J_{5,7}=1,5 Hz) ; 8,50 (d, Hₐᵣₒₘ, 1H, J=4,4 Hz).

### PREPARATION 9 : 6-BROMO-3-[2-(PYRIDIN-4-YL)ETHYL]OXAZOLO[4,5-b]PYRIDIN-2(3H)-ONE

En procédant de la même façon que pour la synthèse du composé de la préparation 8, mais en utilisant la 4-vinylpyridine à la place de la 2-vinylpyridine, on obtient le composé du titre avec un rendement de 65 %. 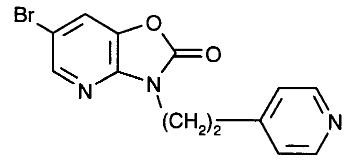
F : 139-141°C
IR (KBr) : 1780 cm⁻¹ (CO carbamate)
RMN ¹H (CDCl₃), δ (ppm) : 3,17 (dd, CH₂, 2H, J₁=8,1 Hz, J₂=7,4 Hz) ;
4,19 (dd, NCH₂, 2H, J₁=8,1 Hz, J₂=7,4 Hz) ; 7,17 (d, Hₐᵣₒₘ, 2H, J=5,9 Hz) ;
7,55 (d, H₇, 1H, J_{5,7}=1,5 Hz) ; 8,16 (d, H₅, 1H, J_{5,7}=1,5 Hz) ; 8,51 (d, Hₐᵣₒₘ, 2H, J=5,9 Hz).

### PREPARATION 10 : 1-METHYLPYRROLO[2,3-b]PYRIDINE

La pyrrolo[2,3-b]pyridine (2,00 g, 16,93 mmol) est dissoute dans le diméthylformamide (15,0 ml), sous atmosphère d'argon. On additionne à 0°C sur une période de 30 minutes l'hydrure de sodium (60 % dans l'huile) (0,96 g, 40,0 mmol, 1,5 eq). Après 30 minutes d'agitation à 0°C, l'iodométhane (1,49 ml, 24,02 mmol, 1,5 eq) est additionné goutte à goutte. Après retour à température ambiante, le milieu réactionnel est laissé sous agitation pendant 1 heure. Le diméthylformamide est évaporé sous pression réduite, le produit est repris avec de l'eau et extrait avec du dichlorométhane. Une purification sur colonne de silice (éther de pétrole/acétate d'éthyle 7/3) permet d'isoler le composé du titre avec un rendement de 99 % sous forme d'une huile. 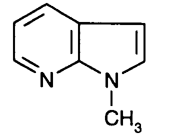
IR (film) : 1597 cm⁻¹ (C=C, Ar)
RMN ¹H (CDCl₃), δ (ppm) : 3,85 (s, 3H, CH₃) ; 6,40 (d, 1H, H-3, J₃₋₂ = 3,3 Hz) ; 7,01 (dd, 1H, H-5, J₅₋₄ = 7,4 Hz, J₅₋₆=5,2 Hz) ; 7,13 (d, 1H, H-2, J₂₋₃=3,3 Hz) ; 7,85 (d, 1H, H₄, J₄₋₅=7,4 Hz) ; 8,29 (d, 1H, H₆, J₆₋₅=5,2 Hz)

### PREPARATION 11 : 1,3-DIHYDRO-1-METHYL-5-BROMO-2H-PYRROLO[2,3-b] PYRIDIN-2-ONE

### Stade 1 : 1,3-dihydro-3,3,5-tribromo-1-méthyl-2H-pyrrolo[2,3-b]pyridin-2-one

- **Méthode A :**
   A une solution du composé de la préparation 10 (5,59 g, 42,3 mmol) dans le tert-butanol (80 ml) est additionné le perbromate de pyridinium (40,58 g, 127,0 mmol, 3,0 eq). Le milieu est agité à température ambiante pendant 2 heures. Les solvants sont concentrés par évaporation sous pression réduite, le produit brut est repris avec de l'eau puis extrait avec de l'acétate d'éthyle. Après évaporation on obtient la 1,3-dihydro-3,3,5-tribromo-1-méthyl-*2H*-pyrrolo[2,3-b]pyridine-2-one avec un rendement de 93 %.
   12,46 g (40,7 mmol) de la 1,3-dihydro 3,3,5-tribromo 1-méthyl-*2H*-pyrrolo[2,3-b]pyridin-2-one ainsi obtenu sont dissous dans 50 ml de diméthylformamide. Après addition goutte à goutte de brome (4,17 ml, 81,4 mmol, 2 eq), le milieu réactionnel est laissé sous agitation pendant 15 heures à température ambiante. Après évaporation du solvant sous pression réduite le produit est repris avec de l'eau puis extrait au dichlorométhane. Le solvant, une fois évaporé, le solide orangé est lavé avec de l'éther de pétrole. Après séchage on obtient le composé du titre avec un rendement de 85 %.
- **Méthode B :**
   La 1-méthylpyrrolo[2,3-b]pyridine de la préparation 10 (2,00 g, 15,1 mmol) est dissoute dans le tert-butanol (132 ml). Une quantité équivalente d'eau (132 ml) est ajoutée lentement. Le brome (9,28 ml, 128,2 mmol 12,0 eq) est additionné goutte à goutte à l'aide d'une ampoule à brome. Après 24 heures d'agitation à température ambiante, le tert-butanol est éliminé par évaporation sous pression réduite. Le mélange est repris avec une solution de d'hydrogénocarbonate de sodium jusqu'à pH neutre, puis filtré. Après séchage on obtient le composé du titre avec un rendement de 91 %.

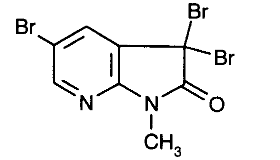

F = 210°C
IR (KBr) : 1747 cm⁻¹ (C=O)
RMN ¹H (CDCl₃) : δ (ppm) : 3,28 (s, 3H, CH₃) ; 3,55 (s, 2H, CH₂), 7,59 (s, 1H, H₄) ; 8,25 (s, 1H, H₆)

### Stade 2 : 1,3-dihydro-5-bromo-1-méthyl-2H-pyrrolo[2,3-b]pyridin-2-one

Le composé obtenu au stade 1 (0,327 g, 0,85 mmol) est dissous dans l'acide acétique (8 ml). On additionne à température ambiante et sous argon du zinc (4,3 g, 8,5 mmol, 10 eq). Après 30 minutes d'agitation à la même température, le milieu réactionnel est filtré puis évaporé sous pression réduite. Le produit brut est extrait avec de l'acétate d'éthyle à pH neutre puis purifié sur colonne de silice (éther de pétrole/acétate d'éthyle 7/3). Le composé du titre est obtenu avec un rendement de 98 %. 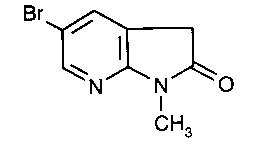
F = 149°C
IR (KBr) : 1713 cm⁻¹ (C=O)
RMN ¹H (CDCl₃), δ (ppm) : 3,28 (s, 3H, CH₃) ; 3,55 (s, 2H, CH₂), 7,59 (s, 1H, H₄) ; 8,25 (s, 1H, H₆).

### PREPARATION 12 : 1,3-DIHYDRO-5-BROMO-3,3-DIMETHYL-2H-PYRROLO[2,3-b] PYRIDIN-2-ONE

Sous argon, dissoudre 3 g (13,21 mmol) du composé de la préparation 11 dans 40 ml de tétrahydrofurane anhydre. Refroidir dans un bain de glace et additionner 793 mg (33,03 mmol) d'hydrure de sodium. Après 30 min. sous agitation, ajouter lentement 2,06 ml (4,69 g, 33,03 mmol) d'iodure de méthyle. Laisser revenir lentement à température ambiante pendant 1 heure. Evaporer le solvant. Reprendre le résidu à l'eau et extraire au dichlorométhane. Sécher la phase organique sur sulfate de magnésium.
Après purification sur colonne de silice, on obtient 2,09 g de produit du titre avec un rendement de 62 %. 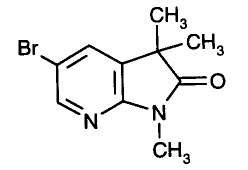
F : 93-94°C
RMN ¹H (CDCl₃) ; δ (ppm) : 1,41 (s, 6H, 2 x CH₃) ; 3,27 (s, 3H, CH₃), 7,50 (d, 1H, H₄) ; 8,23 (d, 1H, H₆).

### PREPARATION 13 : 3-BENZYL-6-BROMO-OXAZOLO[4,5-b]PYRIDIN-2(3H)-ONE

On additionne 215 mg (1,0 mmol) de 6-bromooxazolo[4,5-b]pyridin-*2(3H)*-one à une solution d'éthylate de sodium préparée à partir de 6 ml d'éthanol anhydre et 28 mg (1,2 mmol) de sodium. Après 1 heure d'agitation à température ambiante puis mise à sec sous pression réduite, le résidu est repris dans 6 ml de N, N-diméthyl formamide et le bromure de benzyle ajouté goutte à goutte. Après 2 heures de chauffage au reflux, refroidissement et mise à sec sous pression réduite, le résidu est repris dans l'eau et extrait au dichlorométhane. Le produit brut obtenu par mise à sec est purifié par chromatographie sur colonne de silice (éluant : dichlorométhane).
Rendement 68 %. 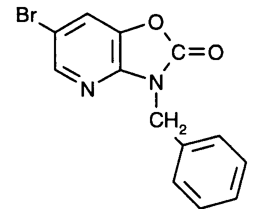
F = 78-80°C
IR (KBr) : 1780 cm⁻¹ (CO carbamate)
RMN ¹H (CDCl₃) : δ (ppm) : 5,12 (s, NCH₂, 2H) ; 7,27-7,39 (m, Hₐᵣₒₘ, 3H) 7,47-7,51 (m, Hₐᵣₒₘ, 2H) 7,54 (d, H₇, 1H, J_{5,7}=2,2 Hz) 8,20 (d, H₅, 1H, J_{5,7}=2,2 Hz)

### PREPARATION 14 : 6-BROMOMETHYL-3-METHYLOXAZOLO[4,5-b]PYRIDIN-2(3H)-ONE

### Stade 1 : 3,6-diméthyloxazolo[4,5-b]pyridin-2(3H)-one

- **Méthode A :**
   La 6-bromo-3-méthyloxazolo[4,5-b]pyridin-*2(3H)*-one de la préparation 2 (100 mg ; 0,45 mmol) est dissoute dans l'hexaméthylphosphoramide (15 ml). Le tétraméthylétain (0,6 ml ; 4,5 mmol) et la tétrakis(triphénylphosphine)palladium (500 mg ; 0,45 mmol) sont successivement ajoutés dans la solution. Le chauffage à reflux est maintenu pendant 4 heures. La solution est reprise avec de l'eau et extraite avec du dichlorométhane. La phase organique est séchée sur sulfate de magnésium, filtrée puis le solvant est évaporé sous pression réduite. La 3,6-diméthyloxazolo[4,5-b]pyridin-*2(3H)*-one est purifiée par chromatographie sur gel de silice (éluant : dichlorométhane). Le rendement obtenu est de 75 %.
- **Méthode B :**
   La 6-bromo-3-méthyloxazolo[4,5-b]pyridin-*2(3H)*-one de la préparation 2 (414 mg ; 1,8 mmol) est dissoute dans le N,N-diméthylformamide (5 ml). Le tétraméthylétain (0,3 ml ; 2,2 mmol), le chlorure de lithium (229 mg ; 5,4 ml) et le tetrakis(triphénylphosphine)palladium (62 mg ; 0,1 mmol) sont successivement ajoutés dans la solution. Le chauffage à reflux est maintenu pendant 8 heures. Lorsque la solution est refroidie et le solvant évaporé sous pression réduite, le résidu est repris avec de l'eau et extrait avec du dichlorométhane. La phase organique est séchée sur sulfate de magnésium, filtrée puis le solvant est évaporé. La 3,6-diméthyloxazolo[4,5-b]pyridin-*2(3H)*-one est purifiée par chromatographie sur gel de silice (éluant : dichlorométhane). Le rendement obtenu est de 66 %.
- **Méthode C :**
   La 6-bromo 3-méthyloxazolo[4,5-b]pyridin-*2(3H)*-one de la préparation 2 (414 mg ; 1,8 mmol) est dissoute dans le toluène (10 ml). Le tétraméthylétain (0,3 ml ; 2,2 mmol), le bis(triphénylphosphine)dichlorure de palladium (70 mg ; 0,1 mmol) sont successivement ajoutés dans la solution. Le chauffage à reflux est maintenu pendant 8 heures. Lorsque la solution est refroidie et le solvant évaporé sous pression réduite, le résidu est repris avec de l'eau et extrait avec du dichlorométhane. La phase organique est séchée sur sulfate de magnésium, filtrée puis le solvant est évaporé. La 3,6-diméthyloxazolo[4,5-b]pyridin-*2(3H)*-one est purifiée par chromatographie sur gel de silice (éluant : dichlorométhane). Le rendement obtenu est de 72%.
   F : 114-116°C
   IR (KBr) : 1780 cm⁻¹ (CO carbamate)

### Stade 2 : 6-bromométhyl-3-méthyloxazolo[4,5-b]pyridin-2(3H)-one

La 3,6-diméthyloxazolo[4,5-b]pyridin-*2(3H)*-one obtenue précédemment au stade 1 (0,50 g ; 3,05 mmol) est dissoute dans du tétrachlorure de carbone distillé (50 ml), on ajoute alors le N-bromosuccinimide (597 mg ; 3,35 mmol) ainsi qu'une quantité catalytique de peroxyde de benzoyle. le chauffage à reflux est maintenu pendant 3 heures. Après refroidissement de la solution puis filtration du succinimide formé, le solvant est évaporé sous pression réduite. Le produit du titre est purifié par flash chromatographie sur gel de silice (éluant : dichlorométhane). Le rendement obtenu est de 83 %. 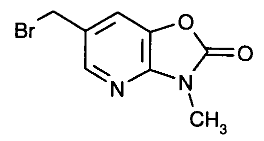
F : 105-106°C
IR (KBr) : 1785 cm⁻¹ (CO carbamate)
RMN ¹H (CDCl₃), δ (ppm) : 3,49 (s, NCH₃, 3H) ; 4,53 (s, CH₂, 2H) ; 7,46 (d, H₇, 1H, J_{5,7}=1,5 Hz) ; 8,13 (d, H₅, 1H, J_{5,7}=1,5 Hz)

### PREPARATION 15 : 3-METHYL-6-[2-(p-TOLUENESULFONYLOXY)ETHYL]OXAZOLO[4,5-b]PYRIDIN-2(3H)-ONE

### Stade 1 : 3-méthyl 6-vinyloxazolo[4,5-b]pyridin-2(3H)-one

On réalise le protocole de la méthode C du stade 1 de la préparation 14, mais en remplaçant le tétraméthylétain par du tributylvinylétain. Le rendement d'obtention de la 3-méthyl-6-vinyloxazolo[4,5-b]pyridin-*2(3H)*-one est de 86 %.
F : 123-125°C
IR (KBr) : 1790 cm⁻¹ (CO carbamate)

### Stade 2 : 6-(2-hydroxyéthyl)-3-méthyloxazolo[4,5-b]pyridin-2(3H)-one

Une solution d'hydrure de bore (1M dans le tétrahydrofurane) (5 ml ; 5 mmol) est refroidie à l'aide d'un bain glace-sel, puis on ajoute lentement du 2,3-diméthyl-1-butène (0,6 ml ; 5 mmol). La température de la solution est amenée à 0°C, l'agitation est maintenue 2 heures supplémentaires à cette même température. La 3-méthyl-6-vinyloxazolo[4,5-b]pyridin-*2(3H)*-one (1,14 mg ; 5 mmol) dissoute dans du tétrahydrofurane (20 ml) est additionnée à la solution de thexylborane précédemment préparée. On laisse sous agitation pendant 2 heures à 0°C, puis une solution aqueuse d'hydroxyde de sodium à 10 % (2,40 ml) ainsi que de l'eau oxygénée (2,00 ml) sont successivement ajoutés. Après une heure d'agitation à température ambiante et évaporation du solvant sous pression réduite, le résidu est repris avec de l'eau. L'extraction est réalisée à l'aide de dichlorométhane ; la phase organique est séchée sur sulfate de magnésium, filtrée puis le solvant est évaporé sous pression réduite. Après purification par flash chromatographie sur gel de silice (éluant : dichlorométhane/méthanol : 95/5), on obtient la 6-(2-hydroxyéthyl) 3-méthyloxazolo[4,5-b]pyridin-*2(3H)*-one avec un rendement de 78 %.
F : 142-144°C
IR (KBr) : 3400-3100 cm⁻¹ (OH), 1775 cm⁻¹ (CO carbamate)

### Stade 3 : 3-méthyl-6-[2-(p-toluènesulfonyloxyléthyl]oxazolo[4,5-b]pyridin-2(3H)-one

La 6-(2-hydroxyéthyl)-3-méthyloxazolo[4,5-b]pyridin-*2(3H)*-one obtenue au stade 2 (194 mg ; 1 mmol) est dissoute dans du dichlorométhane (5 ml). La solution est refroidie à 0°C à l'aide d'un bain glace-sel puis on ajoute le chlorure de tosyle (286 mg ; 1,5 mmol) ainsi que la triéthylamine (0,4 ml ; 3 mmol). L'agitation est maintenue pendant 48 heures en laissant remonter à température ambiante. L'hydrolyse est effectuée en ajoutant de l'eau au milieu réactionnel, l'extraction est ensuite réalisée à l'aide de dichlorométhane. La phase organique est séchée sur sulfate de magnésium, filtrée puis le solvant est évaporé sous pression réduite. Le produit du titre est purifié par chromatographie sur gel de silice (éluant : dichlorométhane). Le rendement obtenu est de 81 %. 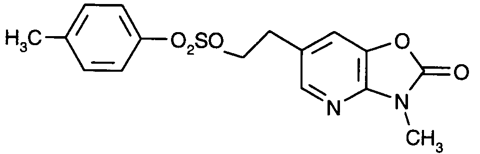
F : 140-143°C
IR (KBr) : 1790 cm⁻¹ (CO carbamate)
RMN ¹H (CDCl₃), δ (ppm) : 2,42 (s, CH₃, 3H) ; 2,97 (t, CH₂, 2H, J=6,6 Hz) ; 3,46 (s, NCH₃, 3H) ; 4,23 (t, CH₂, 2H, J=6,6 Hz ; 7,13 (d, H₇, 1H, J_{5,7}=1,5 Hz) ; 7,27 (d, Hₐᵣₒₘ, 2H, J=8,1 Hz) ; 7,68 (d, Hₐᵣₒₘ, 2H, J=8,1 Hz) ; 7,89 (d, H₅, 1H, J_{5,7}=1,5 Hz).

### PREPARATION 16 : 3-METHYL-6-[3-(p-TOLUENESULFONYLOXY)PROPYL]OXAZOLO[4,5-b]PYRIDIN-2(3H)-ONE

### Stade 1 : 3-méthyl-6-(prop-2-én-3-al-1-yl)oxazolo[4,5-b]pyridin-2(3H)-one

Le mode opératoire est le même que celui utilisé pour le stade 1 méthode C de la préparation 14, le 1-tributylstannyl-3,3-diéthoxyprop-1-ène et le tétrahydrofurane étant respectivement employés à la place du tétraméthylétain et du toluène. Le milieu réactionnel est dissous dans de l'eau puis quelques gouttes d'une solution d'acide chlorhydrique à 10 % sont ajoutées et la solution obtenue agitée pendant 1 heure à température ambiante. Après extraction au dichlorométhane, séchage sur sulfate de magnésium et mise à sec sous pression réduite, le produit brut est purifié par chromatographie sur gel de silice (éluant : dichlorométhane/méthanol : 98/2).
Rendement : 72 % 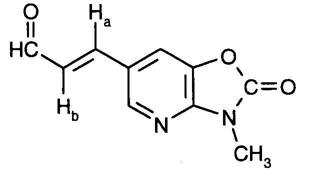
F : 210-212°C
IR (KBr) : 1790 cm⁻¹(CO carbamate), 1680 cm⁻¹ (CO aldéhyde )
RMN ¹H (CDCl₃), δ (ppm) : 3,51 (s, NCH₃, 3H) 6,68 (dd, H_{b}, 1H, J_{a,b}=15,4 Hz, J₂=7,4 Hz ;
7,49 (d, Hₐ, 1H, J_{a,b}=15,4 Hz) ; 7,60 (d, H₇, 1H, J_{5,7}=2,2 Hz) ; 8,29 (d, H₅, 1H, J_{5,7}=2,2 Hz) ; 9,72 (d, CHO, 1H, J=7,4 Hz)

### Stade 2 : 3-méthyl-6-(3-hydroxyprop-1-yl)oxazolo[4,5-b]pyridin-2(3H)-one

La 3-méthyl-6-(prop-2-én-3-al-1-yl)oxazolopyridin-*2(3H)*-one (500 mg) est dissoute dans 35 cm³ de méthanol puis hydrogénée en présence de 50 mg de palladium sur charbon. Après filtration, le produit brut obtenu par mise à sec est purifié par chromatographie sur gel de silice (éluant : dichlorométhane/méthanol : 95/5).
Rendement : 92 % 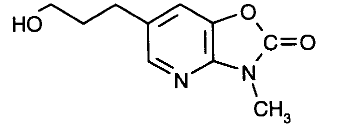
F : 148-149°C
IR (KBr) : 3400-3100 cm⁻¹ (OH), 1770 cm⁻¹ (CO carbamate )
RMN ¹H (CDCl₃ + D₂O), δ (ppm) : 1,84-1,94 (m, CH₂, 2H) ; 2,77 (dd, CH₂, 2H, J₁=8,1 Hz, J₂=7,0 Hz) ; 3,47 (s, NCH₃, 3H) ; 3,70 (dd, CH₂, 2H, J₁=7,0 Hz, J₂=5,9 Hz) ; 7,29 (d, H₇, 1H, J_{5,7}=1,5 Hz) ; 7,98 (d, H₅, 1H, J_{5,7}=1,5 Hz).

### Stade 3 : 3-méthyl-6-[3-(p-toluènesulfonyloxy)propyl]oxazolo[4,5-b]pyridin-2(3H)-one

Le mode opératoire est le même que celui utilisé lors de la synthèse du composé de la préparation 15. La 3-méthyl-6-(3-hydroxypropyl)oxazolo[4,5-b]pyridin-*2(3H)*-one étant employée à la place de la 6-(2-hydroxyéthyl)-3-méthyloxazolo[4,5-b]pyridin-*2(3H)*-one. Le rendement obtenu est de 78 %. 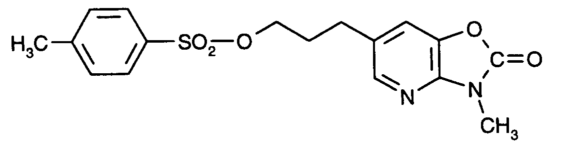
F : 150-152°C
IR (KBr) : 1790 cm⁻¹ (CO carbamate )
RMN ¹H (CDCl₃), δ (ppm) : 1,91-2,03 (m, CH₂, 2H) ; 2,47 (s, CH₃, 3H) ; 2,77 (t, CH₂, 2H, J=7,4 Hz) ; 3,46 (s, NCH₃, 3H) ; 4,05 (t, CH₂, 2H, J=7,4 Hz) ; 7,11 (d, H₇, 1H, J_{5,7}=1,5 Hz) 7,36 (d, Hₐᵣₒₘ, 2H, J=8,1 Hz) 7,79 (d, Hₐᵣₒₘ, 2H, J=8,1 Hz) ; 7,88 (d, H₅, 1H, J_{5,7}=1,5 Hz).

### PREPARATION 17 : 3-METHYL-6-(BROMOACETYL)OXAZOLO[4,5-b]PYRIDIN-2(3H)-ONE

- **Méthode A :**
   La 3-méthyl-6-acétyloxazolo[4,5-b]pyridin-*2(3H)*-one obtenue à l'exemple 1 suivant (500 mg ; 2,6 mmol) est dissoute dans le chloroforme (15 ml), puis le brome (0,2 ml ; 2,6 mmol) est ajouté goutte-à-goutte. La solution est agitée à température ambiante pendant 5 heures. Après avoir évaporé le solvant sous pression réduite, le produit du titre est purifié par chromatographie sur gel de silice (éluant : dichlorométhane). Le rendement obtenu est de 73 %.
- **Méthode B :**
   La 3-méthyl-6-acétyloxazolo[4,5-b]pyridin-*2(3H)*-one décrite (500 mg ; 2,6 mmol) est dissoute dans un mélange acétate d'éthyle/chloroforme : 1/1 (15 ml), puis on additionne en plusieurs fois le bromure de cuivre (1,10 g ; 4,9 mmol). Le milieu réactionnel est porté à reflux pendant 18 heures puis après refroidissement, filtration et mise à sec, le produit du titre est purifié par chromatographie sur gel de silice (éluant : dichlorométhane). Le rendement obtenu est de 70 %.

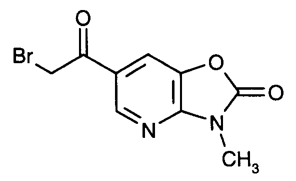

F : 132-134°C
IR (KBr) : 1770 (CO carbamate), 1670 (CO cétone) cm⁻¹
RMN ¹H (CDCl₃), δ (ppm) : 3,54 (s, NCH₃, 3H) ; 4,41 (s, CH₂, 2H) ; 7,97(d, H₇, 1H, J_{5,7}=1,5 Hz) ; 8,81 (d, H₅, J_{5,7}=1,5 Hz)

### PREPARATION 18 : 1,3-DIHYDRO-5-BROMOMETHYL-1-METHYL-2H-PYRROLO[2,3-b] PYRIDIN-2-ONE

### Stade 1 : 1,3-dihydro-1,5-diméthyl-2H-pyrrolo[2,3-b]pyridin-2-one

Le composé de la préparation 11 (0,5 g, 2,2 mmol), et le tétraméthylétain (0,46 ml, 3,35 mmol, 1,5 eq) sont dissous dans le toluène (35 ml). Le tétrakis(triphénylphosphine) palladium (0,077 g, 0,066 mmol, 0,03 eq) et le chlorure de lithium (0,28 g 6,6 mmol, 3 eq) sont additionnés. Le milieu réactionnel est laissé sous agitation, sous argon et à reflux pendant 24 heures. Après élimination du solvant sous pression réduite le milieu est hydrolysé avec de l'eau et extrait à l'acétate d'éthyle. La purification sur colonne de silice (éther de pétrole/acétate d'éthyle, 7 : 3) permet l'obtention du composé du titre sous forme de cristaux rouges avec un rendement de 49 %.
F = 98°C
IR (KBr) : 1718 cm⁻¹ (C=O)

### Stade 2 : 1,3-dihydro-5-bromométhyl-1-méthyl-2H-pyrrolo[2,3-b]pyridin-2-one

Sous atmosphère d'argon, la 1,3-dihydro-1,5-diméthyl-*2H*-pyrrolo[2,3-b]pyridin-2-one du stade 1 (0,1 g, 0,63 mmol) est dissoute dans le tétrachlorure de carbone distillé (12 ml). Le N-bromosuccinimide (0,12 g, 0,66 mmol, 1,05 eq) est additionné. Le milieu réactionnel est porté à relfux, pendant 4h30, puis, après mise à sec, le produit brut est purifié sur colonne de silice (éther de pétrole/acétate d'éthyle, 8:2).
On obtient le composé du titre sous forme d'un solide cristallin avec un rendement de 84 %. 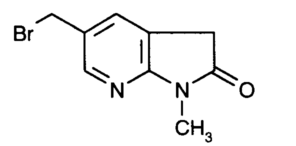
F = 65°C
IR (KBr) : 1705 cm⁻¹ (C=O)
RMN ¹H (CDCl₃), δ (ppm) : 3,30 (s, 3H, CH₃), 3,56 (s, 2H, CH₂), 4,49 (s, 2H, CH₂), 7,55 (s, 1H, H₄), 8,19 (s, 1H, H₆).

### PREPARATION 19 : 1,3-DIHYDRO-5-BROMOACETYL-1-METHYL-2H-PYRROLO[2,3-b] PYRIDIN-2-ONE

Ajouter goutte à goutte 170 mg (1,05 mmole) de brome en solution dans 1 ml d'acide acétique à une solution de 200 mg (1,05 mmole) de 1,3-dihydro-1-méthyl-5-acétyl-*2H*-pyrrolo[2,3-b]pyridin-2-one (obtenue comme décrit dans l'exemple 13 suivant) dans 4 ml d'acide acétique. Après 6 heures de chauffage à 80°C, l'acide acétique est éliminé sous pression réduite et le résidu repris à l'eau puis extrait au dichlorométhane. Le produit brut obtenu est purifié par chromatographie sur gel de silice éluant (acétate d'éthyle/éther de pétrole/dichlorométhane, 1/1/1).
Rendement : 60 %. 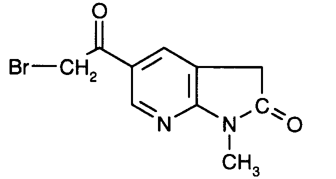
F : 174-175°C
RMN ¹H (CDCl₃+D₂O) : δ (ppm) : 3,36 (s, 3H, N-CH₃) ; 3,62 (s, 2H, CH₂-C=O) ; 4,38 (s, 2H, CH₂Br), 8,07 (s, 1H, H₄) ; 8,88 (s, 1H, H₆)

### PREPARATION 20 : 3-METHYL-6-(2-BROMO-1-HYDROXYETHYL)OXAZOLO[4,5-b] PYRIDIN-2(3H)-ONE

La 3-méthyl-6-bromoacétyloxazolo[4,5-b]pyridin-*2(3H)*-one (1 mmol) obtenue dans la préparation 17 est dissoute dans 15 ml de méthanol anhydre, puis on additionne le borohydrure de sodium (42 mg ; 1,1 mmol). Le milieu réactionnel est agité 5 heures à température ambiante puis hydrolysé. La 3-méthyl-6-(2-bromo-1-hydroxyéthyl)oxazolo[4,5-b] pyridin-*2(3H)*-one est isolée par filtration puis séchée sous vide.
Rendement : 70 %. 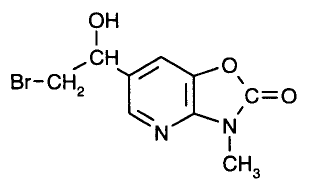
F : 127-129°C
IR (KBr) : 3400-3100 (OH), 1770 cm⁻¹ (CO carbamate)
RMN ¹H (CDCl₃+D₂O) δ (ppm) : 3,48 (s, NCH₃, 3H) ; 3,54 (dd, CHBr, 1H, J₁=10,5 Hz, J₂=8,1 Hz) ; 3,63 (dd, CHBr, 1H, J₁=10,5 Hz, J₂=3,7 Hz) ; 5,00 (dd, CHOH, 1H, J₁=8,1 Hz J₂=3,7 Hz) ; 7,51 (d, H₇, 1H, J_{5,7}=1,5 Hz) ; 8,16 (d, H₅, 1H, J_{5,7}=1,5 Hz)

### PREPARATION 21 : 3-BENZYL-6-BROMOACETYLOXAZOLO[4,5-b]PYRIDIN-2(3H)-ONE

On procède comme pour la préparation 17 mais en remplaçant la 3-méthyl-6-bromooxazol[4,5-b]pyridin-*2(3H)*-one par la 3-benzyl-6-bromo-oxazolo[4,5-b]pyridin-*2(3H)*-one obtenue à la préparation 13.

### PREPARATION 22 : 3-ETHYL-6-BROMOMETHYLOXAZOLO[4,5-b]PYRIDIN-2(3H)-ONE

On procède comme pour les préparations 2 et 14 mais en remplaçant dans l'étape d'alkylation l'iodure de méthyle par l'iodure d'éthyle.

### PREPARATION 23 : 1,3-DIHYDRO-5-BROMOACETYL-1,3,3-TRIMETHYL-2H-PYRROLO[2,3-b]PYRIDIN-2-ONE

En procédant comme pour la préparation 19, mais en remplaçant la 1,3-dihydro-1-méthyl-5-acétyl-*2H*-pyrrolo[2,3-b]pyridin-2-one par la 1,3-dihydro-5-acétyl-1,3,3-triméthyl-*2H*-pyrrolo[2,3-b]pyridin-2-one (exemple 12 suivant), on obtient la 1,3-dihydro-5-bromoacétyl-1,3,3-triméthyl-*2H*-pyrrolo[2,3-b]pyridin-2-one avec un rendement de 59 %. 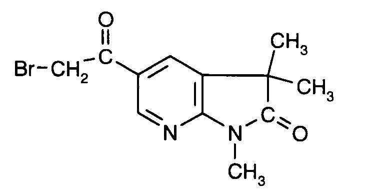
F = 185-186°C
RMN ¹H (CDCl₃) : δ (ppm) : 1,46 (s, 6H, 2 x CH₃) ; 3,22 (s, 3H, N-CH₃) ; 4,38 (s, 2H, CH₂-Br) ; 8,00 (s, 1H, H₄) ; 8,85 (s, 1H, H₆).

### EXEMPLE 1 : 3-METHYL-6-ACETYLOXAZOLO[4,5-b]PYRIDIN-2(3H)-ONE

- **Méthode A : Couplage avec le butylvinyléther**
   La 6-bromo-3-méthyloxazolo[4,5-b]pyridin-*2(3H)*-one de la préparation 2 (0,50 g ; 2,18 mmol) est dissoute dans le N,N-diméthylformamide (5 ml). La triéthylamine (0,44 g ; 4,36 mmol), le butylvinyléther (1,2 g ; 12 mmol), le 1,2-bis(diphénylphosphino)éthane (24 mg; 0,06 mmol) et l'acétate de palladium (II) (12 mg ; 0,054 mmol) sont alors ajoutés dans l'ordre. La solution, sous atmosphère inerte, est portée à reflux pendant 8 heures. Lorsque celleci est refroidie, on hydrolyse à l'aide d'une solution d'acide chlorhydrique à 10 %, l'agitation est maintenue pendant 1 heure. Le N,N-diméthylformamide est évaporé sous pression réduite puis le résidu est repris avec de l'eau, extrait à l'aide du dichlorométhane ; la phase organique est alors séchée sur sulfate de magnésium, filtrée puis le solvant est évaporé. Le produit du titre est purifié par flash chromatographie sur gel de silice (éluant : dichlorométhane/acétate d'éthyle : 8/2).
   Le rendement obtenu est de 90 %.
- **Méthode B : Couplage avec le 1-éthoxy-1-(triméthylstannyl)éthylène**
   La 6-bromo-3-méthyloxazolo[4,5-b]pyridin-*2(3H)*-one de la préparation 2 (700 mg ; 3,05 mmol) est dissoute dans le tétrahydrofurane (20 ml) puis on introduit (3,14 mmoles ; 0,59 ml) de 1-éthoxy-1-(triméthylstannyl)éthylène. Le tétrakis(triphénylphosphine) palladium (180 mg ; 0,15 mmol), le chlorure de lithium (380 mg ; 8,84 mmol) sont alors ajoutés dans l'ordre. La solution, sous atmosphère inerte, est portée à reflux pendant 8 heures. Lorsque celle-ci est refroidie, on hydrolyse à l'aide d'une solution d'acide chlorhydrique à 10 %, l'agitation est maintenue pendant 1 heure. Le tétrahydrofurane est évaporé sous pression réduite puis le résidu est repris avec de l'eau, extrait à l'aide de dichlorométhane ; la phase organique est alors séchée sur sulfate de magnésium, filtrée puis le solvant est évaporé. Le produit du titre est purifié par flash chromatographie sur gel de silice (éluant : dichlorométhane/acétate d'éthyle : 8/2). Le rendement obtenu est de 60 %.

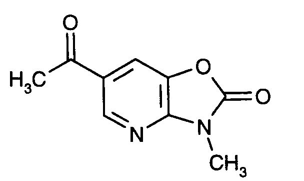

F = 162-164°C (i-PrOH)
IR (KBr) : 1790 cm⁻¹ (CO carbamate) ; 1670 cm⁻¹ (CO cétone)
RMN ¹H (CDCl₃) : δ (ppm) : 2,53 (s, CH₃, 3H) ; 3,63 (s, NCH₃, 3H) ;
7,91 (d, H₇, 1H, J_{5,7}=1,5 Hz) ; 8,72 (d, H₅, 1H, J_{5,7}=1,5 Hz)

### EXEMPLE 2 : 3-BENZYL-6-ACETYLOXAZOLO[4,5-b]PYRIDIN-2(3H)-ONE

Le mode opératoire est le même que celui utilisé lors de la synthèse du composé de l'exemple 1 (couplage avec le 1-éthoxy-1-(triméthylstannyl)éthylène).
La 6-bromo-3-benzyloxazolo[4,5-b]pyridin-*2(3H)*-one de la préparation 13 étant employé à la place de la 6-bromo-3-méthyloxazolo[4,5-b]pyridin-*2(3H)*-one.
Le rendement obtenu est de 75 %. 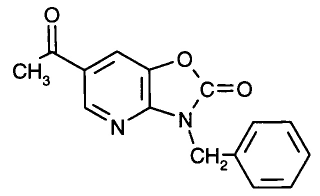
F = 163-164°C
IR (KBr) : 1780 cm⁻¹ (CO carbamate), 1670 cm⁻¹ (CO cétone)
RMN ¹H (CDCl₃) ; δ (ppm) : 2,62 (s, CH₃, 3H) ; 5,12 (s, NCH₂, 2H) ; 7,27-7,78 (m, Hₐᵣₒₘ, 3H) ; 7,49-7,55 (m, Hₐᵣₒₘ, 2H) ; 7,93 (d, H₇, 1H J_{5,7}=1,5 Hz) ; 8,77 (d, H₅, 1H, J_{5,7}=1,5 Hz).

### EXEMPLE 3 : 3-CYANOM ETHYL-6-ACETYLOXAZOLO[4,5-b]PYRIDIN-2(3H)-ONE

Le mode opératoire est le même que celui utilisé lors de la synthèse du composé de l'exemple 1 (couplage avec le 1-éthoxy-1-(triméthylstannyl)éthylène). La 6-bromo-3-(cyanométhyl)oxazolo[4,5-b]pyridin-*2(3H)*-one de la préparation 3 est employée à la place de la 6-bromo-3-méthyloxazolo[4,5-b]pyridin-*2(3H)*-one de la préparation 2. Le rendement obtenu est de 60 %. 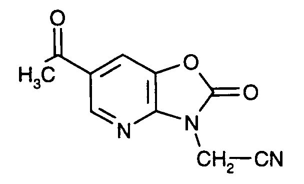
F = 170-172°C
IR (KBr) : 1790 cm⁻¹ (CO carbamate), 1670 cm⁻¹ (CO cétone)
RMN ¹H (CDCl₃) ; δ (ppm) : 2,64 (s, CH₃, 3H) ; 4,85 (s, NCH₂, 2H) ;
8,03 (d, H₇, 1H, J_{5,7}=1,5 Hz) ; 8,79 (d, H₅, 1H, J_{5,7}=1,5 Hz)

### EXEMPLE 4 : 6-ACETYLOXAZOLO[4,5-b]PYRIDIN-2(3H)-ONE

- **Méthode A : Décyanométhylation**
   La 3-cyanométhyl-6-acétyloxazolo[4,5-b]pyridin-*2(3H)*-one de l'exemple 3 (1 g) est dissoute dans de l'éthanol (25 ml) puis on ajoute l'oxyde de platine (250 mg). La solution est agitée à température ambiante sous atmosphère d'hydrogène. Après avoir filtré le catalyseur, le solvant est évaporé sous pression réduite. Le produit du titre est purifié par flash chromatographie sur gel de silice (éluant : dichlorométhane/méthanol : 95/5).
   Le rendement obtenu est de 98 %.
- **Méthode B : Acétylation puis débenzylation**
- ***Stade 1 : 6-acétyl-2-benzyloxyoxazolo[4,5-b]pyridine***
   Le mode opératoire est le même que celui utilisé lors de la synthèse du composé de l'exemple 1 (couplage avec le 1-éthoxy-1-(triméthylstannyl)éthylène), la 2-benzyloxy-6-bromo-oxazolo[4,5-b]pyridine de la préparation 4 étant employée à la place de la 6-bromo-3-méthyloxazolo[4,5-b]pyridin-*2(3H)*-one de la préparation 2.
   Le rendement obtenu est de 78%

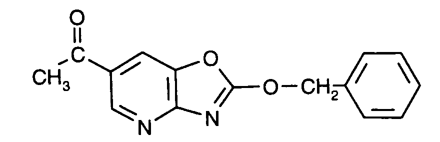

F = 180-182°C
IR (KBr) : 1740 cm⁻¹ -C=N- 1670 cm⁻¹ (CO cétone)
RMN ¹H (CDCl₃) ; δ (ppm) : 2,50 (s, CH₃, 3H) ; 5,48 (s, NCH₂, 2H) ; 7,53 (d, H₇, 1H, J_{5,7}=1,5 Hz) ; 7,35-7,48 (m, Hₐᵣₒₘ, 5H) ; 7,96 (d, H₅, 1H, J_{5,7}=1,5 Hz)

### Stade 2 : 6-acétyloxazolo[4,5-b]pyridin-2(3H)-one

La 6-acétyl-2-benzyloxyoxazolo[4,5-b]pyridine du stade précédent (1 g) est dissoute dans du méthanol (25 ml) puis on ajoute le palladium sur charbon (100 mg). La solution est agitée à température ambiante sous atmosphère d'hydrogène. Après avoir filtré le catalyseur, le solvant est évaporé sous pression réduite. Le produit du titre est purifié par falsh chromatographie sur gel de silice (éluant : dichlorométhane/méthanol : 95/5).
Le rendement obtenu est de 95 %. 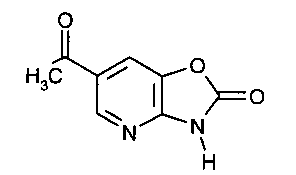
F = 222-224°C (H₂O)
IR (KBr) : 3500-3200 cm⁻¹ (NH), 1750 cm⁻¹ (CO carbamate), 1670 cm⁻¹ (CO cétone)
RMN ¹H (DMSO+D₂O), δ : 2,22 (s, CH₃, 3H) ; 7,87 (s, H₇, 1H) ; 8,66 (s, H₅, 1H) ;

### EXEMPLE 5 : 3-METHYL-6-BENZOYLOXAZOLO[4,5-b]PYRIDIN-2(3H)-ONE

### Stade 1 : 3-méthyl-6-benzyloxazolo[4,5-b]pyridin-2(3H)-one

Le zinc (227 mg ; 3,48 mmol) est mis en suspension dans du tétrahydrofurane (10 ml), on ajoute le 1,2-dibromoéthane (0,22 ml ; 0,2 mmol). Le mélange est chauffé à 60°C pendant 3 minutes, on laisse refroidir la solution jusqu'à ce que la température soit de 35°C, le chlorure de triméthylsilyle (0,06 ml ; 0,5 mmol) est lentement additionné. L'agitation est maintenue pendant 30 minutes puis le bromure de benzyle (0,11 ml ; 0,9 mmol) est rajouté. Attendre 30 minutes supplémentaires avant d'introduire la 6-bromo-3-méthyloxazolo[4,5-b] pyridin-*2(3H)*-one (200 mg ; 0,87 mmol), le tétrakis(triphényl-phosphine)palladium (4 mg). Chauffer à 50°C pendant 20 minutes. Après refroidissement de la solution, le zinc est filtré, le filtrat est repris avec de l'eau, puis on ajoute une solution aqueuse d'acide chlorhydrique à 10 % jusqu'à ce que la phase aqueuse devienne limpide. L'extraction est effectuée à l'aide de dichlorométhane. La phase organique est séchée sur sulfate de magnésium, filtrée puis le solvant est évaporé sous pression réduite. Le produit du titre est purifié par chromatographie sur gel de silice (éluant : dichlorométhane). Le rendement obtenu est de 91 %. 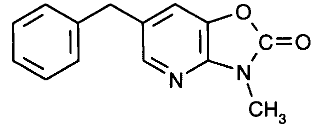
F = 127-129°C
IR (KBr) : 1780 cm⁻¹ (CO carbamate),
RMN ¹H (CDCl₃) : δ (ppm) : 3,46 (s, 3H, NCH₃) ; 4,00 (s, 2H, CH₂), 7,14-7,35 (m, 6H, H₇+5Hₐᵣₒₘ), 8,02 (d, 1H, H₅, J_{5,7}=1,5 Hz)

### Stade 2 : 3-méthyl-6-benzoyloxazolo[4,5-b]pyridin-2(3H)-one

L'oxyde de chrome (VI) (5 mg ; 0,05 mmol) est mis en suspension dans le dichlorométhane (25 ml) puis l'hydrogénopéroxyde de tert-butyle (1,08 ml ; 8 mmol) est ajouté goutte-à-goutte. La 6-benzyl-3-méthyloxazolo[4,5-b]pyridin-2-one du stade 1 (236 mg ; 1 mmol) est lentement additionnée à la solution. Lorsque la couleur du milieu réactionnel est jaune, on rajoute la même quantité d'oxyde de chrome (VI) et d'hydroperoxyde de tert-butyle. L'agitation est maintenue pendant 24 heures supplémentaires. Après avoir filtré sur Célite et évaporé le solvant sous pression réduite, le produit du titre est purifié par chromatographie sur gel de silice (éluant : dichlorométhane).
Le rendement obtenu est de 82 %. 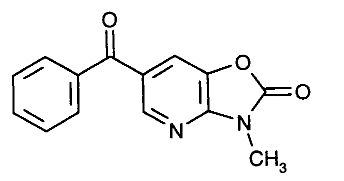
F = 131-133°C (i-PrOH)
IR (KBr) : 1790 cm⁻¹ (CO carbamate), 1635 cm⁻¹ (CO cétone)
RMN ¹H (CDCl₃) ; δ (ppm) : 3,55 (s, NCH₃, 3H) ; 7,48-7,56 (m, Hₐᵣₒₘ, 2H) ; 7,60-7,67 (m, Hₐᵣₒₘ, 1H) ; 7,78 (d, Hₐᵣₒₘ, 2H, J=7,4 Hz) ; 7,89 (d, H₇, 1H, J_{5,7}=1,5 Hz) ; 8,58 (d, H₅, 1H, J_{5,7}=1,5 Hz)

### EXEMPLE 6 : 6-BENZOYLOXAZOLO[4,5-b]PYRIDIN- 2(3H)-ONE

Le mode opératoire est le même que celui utilisé lors de la synthèse du composé de l'exemple 5. La 6-benzyloxazolo[4,5-b]pyridin-*2(3H)*-one est employée à la place de la 6-benzyl-3-méthyloxazolo[4,5-b]pyridin-*2(3H)*-one. Le rendement obtenu est de 50 %. 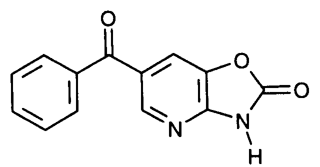
F = 196-198°C
IR (KBr) : 1750 cm⁻¹ (CO carbamate), 1650 cm⁻¹ (CO cétone)
RMN ¹H (DMSO+D₂O) ; δ (ppm) : 7,54-7,62 (m, Hₐᵣₒₘ, 2H) ; 7,67-7,79 (m, Hₐᵣₒₘ, 3H) ; 7,94 (d, H₇, 1H, J_{5,7}=1,5 Hz) ; 8,38 (d, H₅, 1H, J_{5,7}=1,5 Hz)

### EXEMPLE 7 : 6-ACETYL-3-(2-PHENYLETHYL)OXAZOLO[4,5-b]PYRIDIN-2(3H)-ONE

En procédant de la même façon que pour la synthèse du composé de l'exemple 1 (couplage avec le butylvinyléther), mais en remplaçant la 6-bromo-3-méthyloxazolo[4,5-b] pyridin-*2(3H)*-one par la 6-bromo-3-(2-phényléthyl)oxazolo[4,5-b]pyridin-*2(3H)*-one (préparation 5), on obtient le composé du titre avec un rendement de 75 %. 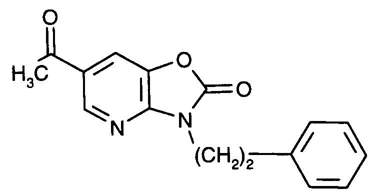
F : 210-212°C
IR (KBr) : 1765 cm⁻¹ (CO carbamate), 1675 cm⁻¹ (CO cétone)
RMN ¹H (CDCl₃) ; δ (ppm) : 2,59 (s, CH₃, 3H) ; 3,13 (dd, CH₂, 2H, J₁=8,1 Hz, J₂=7,4 Hz) ;
4,18 (dd, NCH₂, 2H, J₁=8,1 Hz, J₂=7,4 Hz) ; 7,15-7,28 (m, Hₐᵣₒₘ, 5H) ;
7,88 (d, H₇, 1H, J_{5,7}=1,5 Hz) ; 8,69 (d, H₅, 1H, J_{5,7}=1,5 Hz)

### EXEMPLE 8 : 6-ACETYL-3-(2-CYANOETHYL)OXAZOLO[4,5-b]PYRIDIN-2(3H)-ONE

En procédant de la même façon que pour la synthèse du composé de l'exemple 1 (couplage avec le butylvinyléther), mais en remplaçant la 3-méthyl-6-bromooxazolo[4,5-b]pyridin-*2(3H)*-one par la 3-(2-cyanoéthyl)-6-bromooxazolo[4,5-b]pyridin-*2(3H)*-one (préparation 7), on obtient le composé du titre avec un rendement de 74 %. 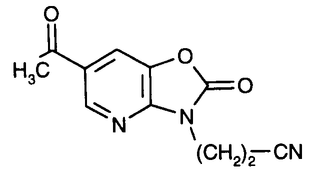
F : 152-154°C
IR (KBr) : 2240 cm⁻¹ (CN), 1780 cm⁻¹ (CO carbamate), 1670 cm⁻¹ (CO cétone)
RMN ¹H (CDCl₃) ; δ (ppm) : 2,65 (s, CH₃, 3H) ; 3,01 (t, CH₂, 2H, J=6,6 Hz) ;
4,81 (t, NCH₂, 2H, J=6,6 Hz) ; 8,01 (d, H₇, 1H, J_{5,7}=1,5 Hz) ; 8,77 (d, H₅, 1H, J_{5,7}=1,5 Hz)

### EXEMPLE 9 : 6-ACETYL-3-[2-(PYRIDIN-2-YL)ETHYL]OXAZOLO[4,5-b]PYRIDIN-2(3H)-ONE

En procédant de la même façon que pour la synthèse du composé de l'exemple 1 (couplage avec le butylvinyléther), mais en remplaçant la 6-bromo 3-méthyloxazolo[4,5-b]pyridin-*2(3H)*-one par la 6-bromo 3-[2-(pyridin-2-yl)éthyl]oxazolo[4,5-b]pyridin-*2(3H)*-one (préparation 8), on obtient le composé du titre avec un rendement de 25 %. 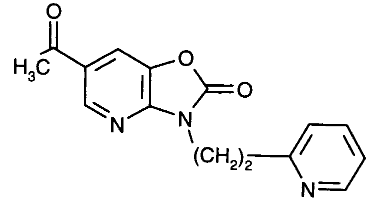
F : 138-140°C
IR (KBr) : 1790 cm⁻¹ (CO carbamate), 1675 cm⁻¹ (CO cétone)
RMN ¹H (CDCl₃), δ : 2,62 (s, CH₃, 3H) ; 3,34 (t, CH₂, 2H, J=7,4 Hz) ; 4,40 (t, NCH₂, 2H, J=7,4 Hz) ; 7,10-7,18 (m, Hₐᵣₒₘ, 2H) ; 7,58 (t, Hₐᵣₒₘ, 1H, J=7,4 Hz) ; 7,91 (d, H₇, 1H, J_{5,7}=1,5 Hz) ; 8,49 (d, Hₐᵣₒₘ, 1H, J=5,2 Hz) ; 8,71 (d, H₅, 1H J_{5,7}=1,5 Hz)

### EXEMPLE 10 : 6-ACETYL-3-[2-(PYRIDIN-4-YL)ETHYL]OXAZOLO[4,5-b]PYRIDIN-2(3H)-ONE

En procédant de la même façon que pour l'exemple 1, mais en utilisant la 6-bromo-3-[2-(pyridin-4-yl)éthyl]oxazolo[4,5-b]pyridin-*2(3H)*-one (préparation 9), on obtient le composé du titre avec un rendement de 50 %. 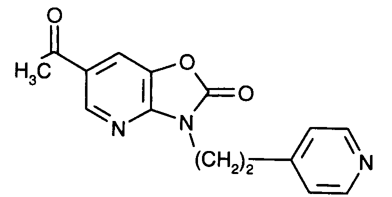
F : 148-150°C
IR (KBr) : 1790 cm⁻¹ (CO carbamate), 1675 cm⁻¹ (CO cétone)
RMN ¹H (CDCl₃) ; δ (ppm) : 2,63 (s, CH₃, 3H) ; 3,20 (t, CH₂, 2H, J=7,4 Hz) ; 4,26 (t, NCH₂, 2H, J=7,4 Hz) ; 7,19 (d, Hₐᵣₒₘ, 2H, J=5,9) ; 7,94 (d, H₇, 1H, J_{5,7}=1,5 Hz) ; 8,52 (d, Hₐᵣₒₘ, 2H, J=5,9 Hz) ; 8,72 (d, H₅, 1H, J_{5,7}=1,5 Hz)

### EXEMPLE 11 : 3-METHYL-6-PROPIONYLOXAZOLO[4,5-b]PYRIDIN-2(3H)-ONE

En procédant de la même façon que pour l'exemple 1 (couplage avec le butylinyléther), mais en remplaçant le butylvinyléther par l'éthyl-1-propényléther, on obtient le composé du titre avec un rendement de 40 %. 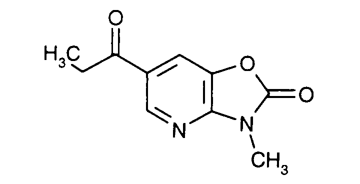
F : 110-112°C
IR (KBr) : 1800 cm⁻¹ (CO carbamate), 1670 cm⁻¹ (CO cétone)
RMN ¹H (CDCl₃), δ : 1,26 (t, CH₃, 3H, J=7,4 Hz) ; 3,02 (m, CH₂, 2H) ; 3,55 (s, NCH₃, 3H) ; 7,98 (d, H₇, J_{5,7}=1,5 Hz) ; 8,79 (d, H₅, J_{5,7}=1,5 Hz)

### EXEMPLE 12 : 1,3-DIHYDRO-1,3,3-TRIMETHYL-5-ACETYL-2H-PYRROLO[2,3-b]PYRIDIN-2-ONE

Sous argon, mettre en suspension 482 mg (11,37 mmol) de chlorure de lithium et 90 mg (7,84 10⁻⁵ mol) de tétrakis(triphénylphosphine)palladium dans 10 ml de toluène anhydre. Ajouter à cette suspension une solution de 1 g (3,92 mmol) de 1,3-dihydro-1,3,3-triméthyl 5-bromo-*2H*-pyrrolo[2,3-b]pyridin-2-one et de 1,46 mg (4,04 mmol) de (1-éthoxyvinyl)tributylétain dans 20 ml de toluène anhydre. Après 5 heures à reflux, évaporer le solvant et reprendre le résidu par un mélange dioxane/acide chlorhydrique 10 %, 1:1 (30 ml). Après 30 mn sous agitation à température ambiante, évaporer le dioxane. Filtrer les sels d'étain obtenus sur célite. Extraire le filtrat au dichlorométhane et sécher sur sulfate de magnésium. Après purification sur colonne de silice on obtient 780 mg de produit du titre avec un rendement de 90 %. 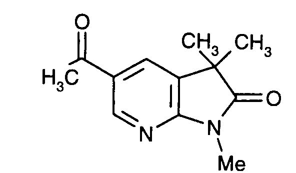
F : 99-100°C
RMN ¹H (CDCl₃) ; δ (ppm) : 1,43 (s, 6H, 2 x CH₃)) ; 2,62 (s, 3H, CH₃-C=O) ; 3,35 (s, 3H, CH₃) ; 8,00 (d, 1H, H₄) , 8,29 (d, 1H, H₆)

### EXEMPLE 13 : 1,3-DIHYDRO-1-METHYL-5-ACETYL-2H-PYRROLO[2,3-b]PYRIDIN-2-ONE

En procédant de la même façon que pour la synthèse du composé de l'exemple 12 mais en remplaçant la 1,3-dihydro-1,3,3-triméthyl-5-bromo-*2H*-pyrrolo[2,3-b]pyridin-2-one par la 1,3-dihydro-1-méthyl-5-bromo-*2H*-pyrrolo[2,3-b]pyridine-2-one, on obtient le composé du titre avec un rendement de 84 %. 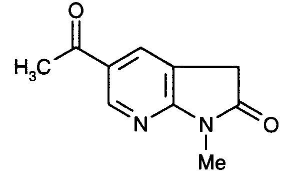
IR : 1705, 1670 cm⁻¹
RMN ¹H (CDCl₃) ; δ (ppm) : 2,58 (s, 3H, CH₃-C=O) ; 3,32 (s, 3H, CH₃) ; 3,58 (s, 2H, CH₂) ; 8,04 (s, 1H₄) ; 8,78 (s, 1H, H₆)

### EXEMPLE 14 : 6-(1-PHENYL-1-HYDROXYMETHYL)-3-METHYLOXAZOLO[4,5-b]PYRIDIN-2(3H)-ONE

Ajouter 42 mg (1,1 mmol) de borohydrure de sodium à une solution préalablement préparée de 254 mg (1 mmol) de 6-benzoyl-3-méthyloxazolo[4,5-b]pyridin-*2(3H)*-one dans 20 ml de méthanol anhydre. Mettre à sec le milieu réactionel sous pression réduite après 5 heures d'agitation à température ambiante puis reprendre le résidu par de l'eau et isoler le produit formé par filtration, on obtient le produit du titre avec un rendement de 84 %.

### EXEMPLE 15 : 5-BENZOYL-1-METHYL-1,3-DIHYDRO-2H-PYRROLO[2,3-b]PYRIDIN-2-ONE

### Stade 1 : 5-benzyl-1-méthyl-1,3-dihydro-2H-pyrrolo[2,3-b]pyridin-2-one

En procédant comme pour le stade 1 de l'exemple 5 mais en remplaçant la 6-bromo-3-méthyloxazolo[4,5-b]pyridin-*2(3H)*-one par la 1,3-dihydro-5-bromo-1-méthyl-*2H*-pyrrolo[2,3-b]pyridin-2-one on obtient le composé du titre avec un rendemnt de 20 %. 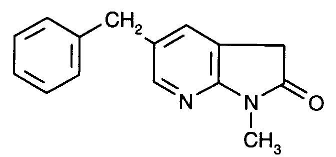
RMN ¹H (CDCl₃) ; δ (ppm) : 3,30 (s, 3H, N-CH₃); 3,48 (s, 2H, CH₂-C=O) ; 3,94 (s, 2H, CH₂-Ph) ; 7,15-7,33 (m, 6H, Hₐᵣₒₘ + H₄) ; 8,08 (s, 1H, H₆).

### Stade 2 : 5-benzyl-3,3-dibromo-1-méthyl-1,3-dihydro-2H-pyrrolo[2,3-b]pyridin-2-one

Ajouter 60 mg de N-bromosuccinimide et une pointe de spatule de peroxyde de dibenzoyle à une solution préalablement préparée de 40 mg du composé du stade 1 dans le tétrachlorure de carbone. Chauffer à reflux pendant 1 heure pour mettre à sec et purifier le produit brut obtenu par chromatographie sur colonne de silice (éluant : éther de pétrole/acétate d'éthyle : 7/3).
On obtient le produit du titre avec un rendement de 72 %. 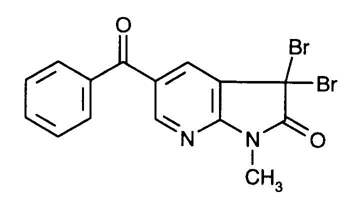
RMN ¹H (CDCl₃) ; δ (ppm) : 3,40 (s, 3H, CH₃) ; 7,54 (t, 2H, J=7,35 Hz, Hₐᵣₒₘ) ; 7,65 (d, 1H, J=7,35 Hz, Hₐᵣₒₘ) ; 7,80 (d, 2H, J=7,35 Hz, Hₐᵣₒₘ) 8,33 (d, 1H, J=2,2 Hz, H₄) ; 8,66 (d, 1H, J=2,2 Hz, H₆).

### Stade 3 : 5-benzoyl-1-méthyl-1,3-dihydro-2H-pyrrolo[2,3-b]pyridin-2-one

Ajouter une pointe de spatule de zinc à une solution de 40 mg du composé du stade 2 dans l'acide acétique. Après 30 minutes d'agitation, mettre à sec sous pression réduite, reprendre à l'eau et extraire au dichlorométhane. Le produit brut obtenu par mise à sec est purifié par chromatographie sur colonne de silice (éluant : éther de pétrole/acétate d'éthyle, 6/4).
On obtient le produit du titre sous forme d'huile avec un rendement de 60 %. 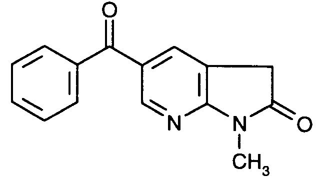
RMN ¹H (CDCl₃) ; δ (ppm) : 3,37 (s, 3H, N-CH₃) ; 3,64 (s, 2H, CH₂=O) ; 7,53 (t, 2H, J=7,35 Hz, Hₐᵣₒₘ) ; 7,64 (d, 1H, J=7,35 Hz, Hₐᵣₒₘ), 7,79 (d, 2H, J=7,35 Hz, Hₐᵣₒₘ) ; 8,02 (d, 1H, J=1,2 Hz, H₄) ; 8,63 (d, 1H, J=1,2 Hz, H₆).

### EXEMPLE 16 : 3-METHYL-6-[(4-PHENYLPIPERAZIN-1-YL)METHYL]OXAZOLO[4,5-b]PYRIDIN-2(3H)-ONE

La 6-bromométhyl-3-méthyloxazolo[4,5-b]pyridin-*2(3H)*-one de la préparation 14 (1g ; 4,1 mmol) est dissoute dans le 1,4-dioxane (25 ml). La N-phénylpipérazine (0,70 g ; 4,3 mmol) et la triéthylamine (0,62 g ; 6,2 mmol) sont successivement ajoutées au milieu réactionnel. La solution est agitée pendant 5 heures à température ambiante et sous atmosphère inerte. Après avoir évaporé le solvant sous pression réduite, le résidu est repris avec de l'eau puis extrait avec du dichlorométhane ; la phase organique est séchée sur sulfate de magnésium, filtrée puis le solvant est évaporé. Le produit du titre est purifié par chromatographie sur gel de silice (éluant : dichlorométhane/méthanol : 98/2). Le rendement obtenu est de 96 %. 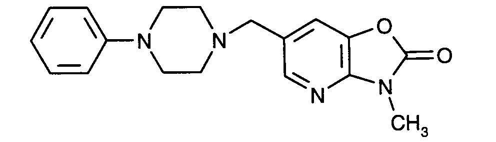
F = 130-132°C (i-PrOH)
IR (KBr) : 1775 cm⁻¹ (CO carbamate)
RMN ¹H (CDCl₃), δ (ppm) : 2,61 (dd, 2xCH₂pipér., 4H, J₁=5,2 Hz J₂=4,4 Hz) ; 3,19 (dd, 2xCH₂pipér., 4H, J₁=5,2 Hz J₂=4,4 Hz) ; 3,48 (s, NCH₃, 3H) ; 3,58 (s, CH₂, 2H) ; 6,86 (t, Hₐᵣₒₘ, 1H, J=7,3 Hz) ; 6,92 (d, Hₐᵣₒₘ, 2H, J=8,8, Hz) ; 7,24 (d, Hₐᵣₒₘ, 2H, J=8,8 Hz) ; 7,51 (d, H₇, 1H, J_{5,7}=1,8 Hz) ; 8,05 (d, H₅, 1H, J_{5,7}=1,8 Hz).
MS (IC/NH₃) : m/z : 325 (M+1).

### EXEMPLE 17 : 3-METHYL-6-(MORPHOLINOMETHYL)OXAZOLO[4,5-b]PYRIDIN-2(3H)-ONE

Le mode opératoire est le même que celui utilisé lors de la synthèse du composé de l'exemple 16. La morpholine est employée à la place de la N-phénylpipérazine. Le rendement obtenu est de 90 %. 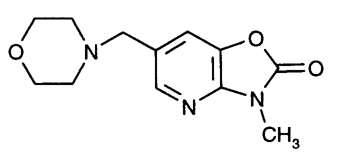
F = 143-145°C (i-PrOH)
IR (KBr) : 1775 cm⁻¹ (CO carbamate)
RMN ¹H (CDCl₃), δ (ppm) : 2,46 (dd, 2xCH₂morph., 4H, J₁=5,2 Hz J₂=4,3 Hz) ; 3,49 (s, NCH₃, 3H) ; 3,52 (s, CH₂, 2H) ; 3,71 (dd, 2xCH₂morph., 4H, J₁=5,2 Hz J₂=4,3 Hz) ; 7,50 (d, H₇, 1H, J_{5,7}=1,8 Hz) ; 8,03 (d, H₅, 1H, J_{5,7}=1,8 Hz)
MS (IC/NH₃) : m/z = 250 (M+1)

### EXEMPLE 18 : 3-METHYL-6-[2-(4-PHENYLPIPERAZIN-1-YL)ETH-1-YL]OXAZOLO[4,5-b]PYRIDIN-2(3H)-ONE

La 3-méthyl-6-[2-(p-toluènesulfonyloxy)éthyl]oxazolo[4,5-b]pyridin-*2(3H)*-one de la préparation 15 (210 mg ; 1,2 mmol) est dissoute dans le 1,4-dioxane (8 ml), la N-phénylpipérazine (0,10 ml ; 1,38 mmol) ainsi que la triéthylamine (0,18 ml ; 1,3 mmol) sont successivement additionnées au milieu réactionnel. L'agitation est maintenue à température ambiante pendant 24 heures. Après avoir évaporé le solvant sous pression réduite, le résidu est repris avec de l'eau. L'extraction est réalisée à l'aide de dichlorométhane, la phase organique est séchée sur sulfate de magnésium, filtrée, puis le solvant est évaporé. Le produit du titre est purifié par chromatographie sur gel de silice (éluant : dichlorométhane/méthanol : 95/5). Le rendement obtenu est de 91 %. 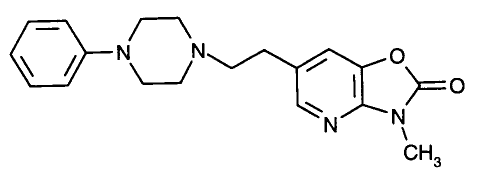
F = 80-82°C (lavage éther)
IR (KBr) : 1790 cm⁻¹ (CO carbamate)
RMN ¹H (CDCl₃), δ (ppm) : 2,64-2,72 (m, CH₂+2xCH₂pipér., 6H) ; 2,88 (dd, CH₂, 2H, J₁=8,0 Hz J₂=7,1 Hz) ; 3,24 (dd, 2xCH₂piper., 4H, J₁=5,6 Hz J₂=4,8 Hz) ; 3,49 (s, NCH₃, 3H) ; 6,88 (t, Hₐᵣₒₘ, 1H, J=7,1 Hz) ; 6,95 (d, Hₐᵣₒₘ, 2H, J=7,9 Hz) ; 7,24-7,32 (m, Hₐᵣₒₘ, 2H) ; 7,35 (d, H₇, 1H, J_{5,7}=1,6, Hz) ; 8,01 (d, H₅, 1H, J_{5,7}=1,6, Hz)

### EXEMPLE 19 : 3-METHYL-6-(2-MORPHOLINOETHYL)OXAZOLO[4,5-b]PYRIDIN-2(3H)-ONE

En procédant comme pour l'exemple 18 mais en remplaçant la phénylpipérazine par la morpholine on obtient la 3-méthyl-6-(2-morpholinoéthyl)oxazolo[4,5-b]pyridin-*2(3H)*-one. 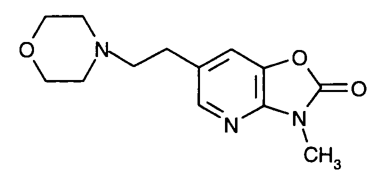
Huile
IR (KBr) : 1790 cm⁻¹ (CO carbamate)
RMN ¹H (CDCl₃), δ (ppm) : 2,50 (dd, 2xCH₂morph., 4H, J₁=5,2 Hz J₂=4,4 Hz) ;
2,59 (t, CH₂, 2H, J=7,3 Hz) ; 2,83 (t, CH₂, 2H, J = 7,3 Hz) ; 3,46 (s, NCH₃, 3H) ; 3,73 (dd, 2xCH₂morph., 4H, J₁=5,2 Hz, J₂=4,4 Hz) ; 7,33 (d, H₇, 1H, J_{5,7}=1,5 Hz) ; 7,97 (d, H₅, J_{5,7}=1,5 Hz).

### EXEMPLE 20 : 3-METHYL-6-[3-(4-PHENYLPIPERAZIN-1-YL)PROP-1-YL]OXAZOLO [4,5-b]PYRIDIN-2(3H)-ONE

Le mode opératoire est le même que celui utilisé lors de la synthèse du composé de l'exemple 18, la 3-méthyl-6-[3-(p-toluènesulfonyloxy)-n-prop-1-yl]oxazolo[4,5-b]pyridin-*2(3H)*-one de la préparation 16 étant employée à la place de la 3-méthyl-6-[2-(p-toluène sulfonyloxy)éthyl]oxazolo[4,5-b]pyridin-*2(3H)*-one. 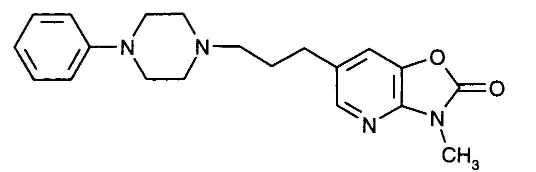
F < 60°C (lavage éther)
IR (KBr) : 1785 cm⁻¹ (CO carbamate)
RMN ¹H (CDCl₃), δ (ppm) : 1,79-1,90 (m, CH₂, 2H) ; 2,41 (t, CH₂, 2H, J=7,4 Hz) ; 2,59 (dd, 2xCH₂pipér., 4H, J₁=5,1 Hz, J₂=4,4 Hz) ; 2,71 (t, CH₂, 2H, J=7,4 Hz) ; 3,21 (dd, 2xCH₂pipér., 4H, J₁=5,1 Hz, J₂=4,4 Hz) ; 3,49 (s, NCH₃, 3H) ; 6,85 (t, Hₐᵣₒₘ, 1H, J=7,4 Hz) ; 6,92 (d, Hₐᵣₒₘ, 2H, J=8,1 Hz) ; 7,22-7,29 (m, H₇+Hₐᵣₒₘ, 3H) ; 7,96 (d, H₅, 1H, J_{5,7}=1,5 Hz)

### EXEMPLE 21 : 3-METHYL-6-(3-MORPHOLINO-N-PROP-1-YL)OXAZOLO[4,5-b] PYRIDIN-2(3H)-ONE

En procédant comme pour l'exemple 20 mais en remplaçant la phénylpipérazine par la morpholine on obtient la 3-méthyl-6-(3-morpholino-n-prop-1-yl)oxazolo[4,5-b]pyridin-*2(3H)*-one avec un rendement de 83 %. 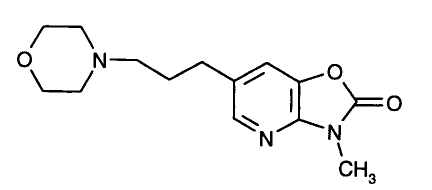
F < 80°C (lavage éther)
IR (KBr) : 1790 cm⁻¹ (CO carbamate)
RMN ¹H(CDCl₃), δ (ppm) : 1,75-1,90 (quint., CH₂, 2H, J=7,4 Hz) ; 2,37 (t, CH₂, 2H, J=7,4 Hz) ; 2,44 (t, 2xCH₂morph., 4H, J=4,4 Hz) ; 2,71 (t, CH₂, 2H, J=7,4 Hz) ; 3,49 (s, NCH₃, 3H) ; 3,74 (dd, 2xCH₂morph., 4H, J=4,4 Hz) ; 7,28 (d, H₇, 1H, J_{5,7}=1,5 Hz) 7,97 (d, H₅, J_{5,7}=1,5 Hz)

### EXEMPLE 22 : 3-METHYL-6-[2-(4-PHENYLPIPERAZIN-1-YL)ACETYL]OXAZOLO[4,5-b] PYRIDIN-2(3H)-ONE

Le mode opératoire est le même que celui utilisé lors de la synthèse du composé de l'exemple 16, la 3-méthyl-6-(bromoacétyl)oxazolo[4,5-b]pyridin-*2(3H)*-one de la préparation 17 étant employée à la place de la 3-méthyl-6-(bromométhyl)oxazolo[4,5-b]pyridin-*2(3H)*-one.
Le rendement obtenu est de 84 %. 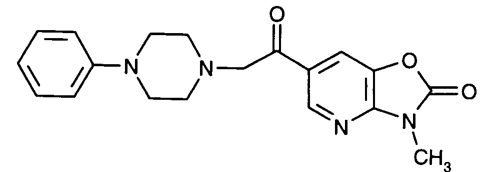
F : 201-203°C (lavage éther)
IR (KBr) : 1775 cm⁻¹ (CO carbamate)
RMN ¹H (CDCl₃), δ (ppm) : 2,75 (dd, 2xCH_{2pipér.}, 4H, J₁=5,1 Hz J₂=4,4 Hz) ; 3,24 (dd, 2xCH_{2pipér.}, 4H, J₁=5,1 Hz J₂=4,4 Hz) ; 3,52 (s, NCH₃, 3H) ; 3,78 (s, CH₂, 2H) ; 6,85 (t, Hₐᵣₒₘ, 1H, J=7,4 Hz) ; 6,92 (d, Hₐᵣₒₘ, 2H, J=8,1 Hz) ; 7,21-7,30 (m, Hₐᵣₒₘ, 2H) ; 8,05 (d, H₇, 1H, J_{5,7}=1,5 Hz) ; 8,99 (d, H₅, J_{5,7}=1,5 Hz)

### EXEMPLE 23 : 3-METHYL-6-(2-MORPHOLINOACETYL)OXAZOLO[4,5-b]PYRIDIN-2(3H)-ONE

En procédant comme pour l'exemple 22 mais en remplaçant la phénylpipérazine par la morpholine, on obtient la 3-méthyl-6-(2-morpholinoacétyl)oxazolo[4,5-b]pyridin-*2(3H)*-one avec un rendement de 86 %. 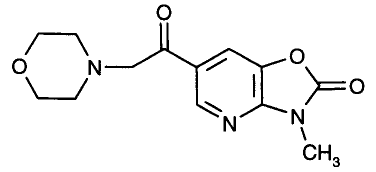
F : 185-187°C (lavage éther)
IR (KBr) : 1775 cm⁻¹ (CO carbamate)
RMN ¹H (CDCl₃), δ (ppm) : 2,57 (dd, 2xCH₂morph., 4H, J₁=5,2 Hz J₂=4,4 Hz) ; 3,51 (s, NCH₃, 3H) ; 3,71 (s, CH₂, 2H) ; 3,73 (dd, 2xCH₂morph., 4H, J₁=5,2 Hz J₂=4,4 Hz) ; 8,01 (d, H₇, 1H, J_{5,7}=1,5 Hz) ; 8,93 (d, H₅, J_{5,7}=1,5 Hz)

### EXEMPLE 24 : 3-METHYL-6-[2-(4-PHENYLPIPERAZIN-1-YL)-1-HYDROXYETHYL] OXAZOLO[4,5-b]PYRIDIN-2(3H)-ONE

- **Méthode A : A partir de la 3-méthyl-6-[2-(4-phénylpipérazin-1-yl)acétyl]oxazolo** **[4,5-b]pyridin-*2(3H)*-one**
   La 3-méthyl-6-[2-(4-phénylpipérazin-1-yl)acétyl]oxazolo[4,5-b]pyridin-*2(3H)*-one de l'exemple 22 (352 mg ; 1 mmol) est dissoute dans du méthanol anhydre (15 ml), puis on ajoute le borohydrure de sodium (42 mg ; 1,1 mmol) à la solution. L'agitation est maintenue à température ambiante pendant 5 heures. Après avoir ajouté de l'eau au milieu réactionnel, le produit du titre formé est filtré puis séché sous vide.
   Le rendement obtenu est de 95 %.
- **Méthode B : A partir de la 3-méthyl-6-(2-bromo-1-hydroxyéthyl)oxazolo[4,5-b] pyridin-*2(3H)*-one**
   On procède comme pour l'exemple 22 mais en remplaçant la 3-méthyl-6-(bromoacétyl)oxazolo[4,5-b]pyridin-*2(3H)*-one par la 3-méthyl-6-(2-bromo-1-hydroxyéthyl) oxazolo[4,5-b]pyridin-*2(3H)*-one de la préparation 20.
   Le rendement obtenu est de 90 %.

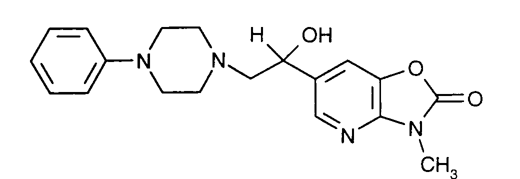

F : 182-184°C (lavage éther)
IR (KBr) : 3400-3100 (OH), 1775 (CO carbamate) cm⁻¹
RMN ¹H (CDCl₃+D₂O), δ (ppm) : 2,47-2,60 (m, NCH₂, 2H)) ; 2,60-2,70 (m, CH₂pipér., 2H) ; 2,88-2,98 (m, CH_{2pipér.}, 2H) ; 3,18-3,33 (m, 2xCH_{2pipér.}, 4H) ; 3,48 (s, NCH₃, 3H) ; 4,83 (dd, CH, 1H, J₁=10,3-4 Hz, J₂=4,4 Hz) ; 6,88 (t, Hₐᵣₒₘ, 1H, J=7,4 Hz) ; 6,94 (d, Hₐᵣₒₘ, 2H, J=8,1 Hz) ; 7,29 (m, Hₐᵣₒₘ, 2H, J=8,1 Hz) ; 7,53 (d, H₇, 1H, J_{5,7}=1,5 Hz) ; 8,10 (d, H₅, J_{5,7}=1,5 Hz)

### EXEMPLE 25 : 3-METHYL-6-[2-MORPHOLINO-1-HYDROXYETHYL]OXAZOLO[4,5-b] PYRIDIN-2(3H)-ONE

- **Méthode A : A partir de l'aminocétone de l'exemple 23**
   Le mode opératoire est le même que celui utilisé lors de la synthèse du composé de l'exemple 24, méthode A.
   Le rendement obtenu est de 98 %.
- **Méthode B : A partir du bromoalcool**
   Le mode opératoire est le même que celui utilisé lors de la synthèse du composé de l'exemple 24, méthode B.
   Le rendement obtenu est de 93 %.

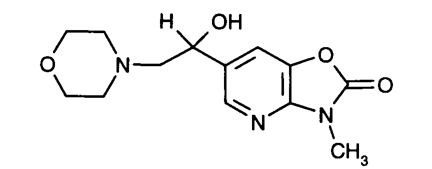

F : 145-147°C (lavage éther)
IR (KBr) : 3400-3100 cm⁻¹ (OH), 1775 cm⁻¹ (CO carbamate)
RMN ¹H (CDCl₃+D₂O), δ (ppm) : 2,36-2,56 (m, NCH₂+CH₂morph., 4H)) ; 2,68-2,78 (m, CH₂morph, 2H) ; 3,48 (s, NCH₃, 3H) ; 3,66-3,80 (m, 2xCH₂morph., 4H) ; 4,76 (dd, CH, 1H, J₁=10,4 Hz J₂=3,9 Hz) ; 7,47 (d, H₇, 1H, J_{5,7}=1,5 Hz) ; 8,04 (d, H₅, J_{5,7}=1,5 Hz)

### EXEMPLE 26 : 3-METHYL-6-[(4-(PYRIMID-2-YL-)PIPERAZIN-1-YL)METHYL]OXAZOLO [4,5-b]PYRIDIN-2(3H)-ONE

En procédant de la même façon que pour l'exemple 16, mais en remplaçant la 4-phénylpipérazine par la 4-(pyrimid-2-yl-)pipérazine, on obtient le composé du titre.

### EXEMPLE 27 : 3-METHYL-6-[(4-METHYLPIPERAZIN-1-YL)METHYL]OXAZOLO[4,5-b] PYRIDIN-2(3H)-ONE

En procédant de la même façon que pour l'exemple 16, mais en remplaçant la 4-phénylpipérazine par la 4-méthylpipérazine, on obtient le composé du titre.

### EXEMPLE 28 : 3-METHYL-6-[2-(4-BENZYLPIPERAZIN-1-YL)ACETYL]OXAZOLO[4,5-b] PYRIDIN-2(3H)-ONE

En procédant de la même façon que pour l'exemple 22, mais en remplaçant la phénylpipérazine par la 4-benzylpipérazine, on obtient le composé du titre.

### EXEMPLE 29 : 3-METHYL-6-{2-[4-(4-FLUOROPHENYL)PIPERAZIN-1-YL]ACETYL} OXAZOLO[4,5-b]PYRIDIN-2(3H)-ONE

En procédant de la même façon que pour l'exemple 22, mais en remplaçant la phénylpipérazine par la 4-(4-fluorophényl)pipérazine, on obtient le composé du titre.

### EXEMPLE 30 : 3-METHYL-6-{2-[4-(3-TRIFLUOROMETHYLPHENYL)PIPERAZIN-1-YL] ACETYL}OXAZOLO[4,5-b]PYRIDIN-2(3H)-ONE

En procédant de la même façon que pour l'exemple 22, mais en remplaçant la phénylpipérazine par la 4-(3-trifluorométhylphényl)pipérazine, on obtient le composé du titre.

### EXEMPLE 31 : 3-METHYL-6-[2-(4-BENZHYDRYLPIPERAZIN-1-YL)-1-HYDROXYETHYL OXAZOLO[4,5-b]PYRIDIN-2(3H)-ONE

En procédant de la même façon que pour l'exemple 24, mais en remplaçant la phénylpipérazine par la 4-benzhydrylpipérazine, on obtient le composé du titre.

### EXEMPLE 32 : 3-METHYL-6-{2-[4-(4,4'-DIFLUOROBENZHYDRYL)PIPERAZIN-1-YL]-1-HYDROXYETHYL}OXAZOLO[4,5-b]PYRIDIN-2(3H)-ONE

En procédant de la même façon que pour l'exemple 24, mais en remplaçant la phénylpipérazine par la 4-(4,4'-difluorobenzhydryl)pipérazine, on obtient le composé du titre.

### EXEMPLE 33 : 3-METHYL-6-[2-(3-AZASPIRO[5,5]UNDECAN-3-YL)ETH-1-YL]OXAZOLO [4,5-b]PYRIDIN-2(3H)-ONE

En procédant de la même façon que pour l'exemple 16, mais en remplaçant la phénylpipérazine par le 3-azaspiro[5,5]undecane, on obtient le composé du titre.

### EXEMPLE 34 : 3-METHYL-6-[2-(3-AZABICYCLO[3,2,2]NONAN-3-YL)ETH-1-YL] OXAZOLO[4,5-b]PYRIDIN-2(3H)-ONE

En procédant de la même façon que pour l'exemple 16, mais en remplaçant la phénylpipérazine par le 3-azabicyclo[3,2,2]nonane, on obtient le composé du titre.

### EXEMPLE 35 : 3-METHYL-6-{2-[4-(PYRID-2-YL)PIPERAZIN-1-YL]ACETYL}OXAZOLO [4,5-b]PYRIDIN-2(3H)-ONE

En procédant de la même façon que pour l'exemple 22, mais en remplaçant la phénylpipérazine par la 4-(pyrid-2-yl)pipérazine, on obtient le composé du titre.

### EXEMPLE 36 : 3-METHYL-6-(2-THIOMORPHOLINOACETYL)OXAZOLO[4,5-b] PYRIDIN-2(3H)-ONE

En procédant de la même façon que pour l'exemple 22, mais en remplaçant la phénylpipérazine par la thiomorpholine, on obtient le composé du titre.

### EXEMPLE 37 : 3-METHYL-6-[2-(N,N-DIPROPYLAMINO)ACETYL]OXAZOLO[4,5-b] PYRIDIN-2(3H)-ONE

En procédant de la même façon que pour l'exemple 22, mais en remplaçant la phénylpipérazine par la N,N-dipropylamine, on obtient le composé du titre.

### EXEMPLE 38 : 3-METHYL-6-(2-ANILINOACETYL)OXAZOLO[4,5-b]PYRIDIN-2(3H)-ONE

En procédant de la même façon que pour l'exemple 22, mais en remplaçant la phénylpipérazine par l'aniline, on obtient le composé du titre.

### EXEMPLE 39 : 3-METHYL-6-[2-(N-BENZYLAMINO)1-HYDROXY-ETHYL)OXAZOLO [4,5-b]PYRIDIN-2(3H)-ONE

En procédant de la même façon que pour l'exemple 24, mais en remplaçant la phénylpipérazine par la N-benzylamine, on obtient le composé du titre.

### EXEMPLE 40 : 3-METHYL-6-[2-(4-NAPHT-1-YL)PIPERAZIN-1-YL-1-HYDROXY-ETHYL) OXAZOLO[4,5-b]PYRIDIN-2(3H)-ONE

En procédant de la même façon que pour l'exemple 24, mais en remplaçant la phénylpipérazine par la 4-(napht-1-yl)pipérazine, on obtient le composé du titre.

### EXEMPLE 41 : 3-METHYL-6-(2-PYRROLIDINOETHYL)OXAZOLO[4,5-b]PYRIDIN-2(3H)-ONE

En procédant de la même façon que pour l'exemple 18, mais en remplaçant la phénylpipérazine par la pyrrolidine, on obtient le composé du titre.

### EXEMPLE 42 : 3-METHYL-6-(3-PIPERIDINO-n-PROP-1-YL)OXAZOLO[4,5-b]PYRIDIN-2(3H)-ONE

En procédant de la même façon que pour l'exemple 20, mais en remplaçant la phénylpipérazine par la pipéridine, on obtient le composé du titre.

### EXEMPLE 43 : 3-METHYL-6-[3-(3-AZABICYCLO[3,3,0]OCT-3-YL)-n-PROP-1-YL] OXAZOLO[4,5-b]PYRIDIN-2(3H)-ONE

En procédant de la même façon que pour l'exemple 20, mais en remplaçant la phénylpipérazine par le 3-azabicyclo[3,3,0]octane, on obtient le composé du titre.

### EXEMPLE 44 : 3-BENZYL-6-[2-(4-PHENYLPIPERAZIN-1-YL)ACETYL]OXAZOLO[4,5-b] PYRIDIN-2(3H)-ONE

En procédant comme pour l'exemple 22 mais en remplaçant la 3-méthyl-6-bromoacétyloxazolo[4,5-b]pyridin-*2(3H)*-one par la 3-benzyl-6-bromoacétyloxazolo[4,5-b] pyridin-*2(3H)*-one de la préparation 21, on obtient le composé du titre.

### EXEMPLE 45 : 3-ETHYL-6-[(4-PHENYLPIPERAZIN-1-YL)METHYL]OXAZOLO[4,5-b] PYRIDIN-2(3H)-ONE

En procédant comme pour l'exemple 16 mais en remplaçant la 3-méthyl-6-bromométhyloxazolo[4,5-b]pyridin-*2(3H)*-one par la 3-éthyl-6-bromoéthyloxazolo[4,5-b] pyridin-*2(3H)*-one de la préparation 22, on obtient le composé du titre.

### EXEMPLE 46: 1,3-DIHYDRO-5-(4-PHENYLPIPERAZIN-1-YLMETHYL)-1-METHYL-2H-PYRROLO[2,3-b]PYRIDIN-2-ONE

La 1,3-dihydro-5-bromométhyl-1-méthyl-*2H*-pyrrolo[2,3-b]pyridin-2-one de la préparation 18 (0,14 g, 0,6 mmol) est dissoute dans le dioxane (20 ml) à température ambiante et sous argon. On additionne successivement, à la même température, la phénylpipérazine (0,1 mln 0,66 mmol, 1,1 eq) et la triéthylamine (0,1 ml, 0,72 mmol, 1,2 eq). Après 1h30 de réaction, le milieu réactionnel est concentré sous pression réduite et le produit est purifié sur colonne de silice (dichlorométhane/méthanol/ 97:3). On obtient le composé du titre sous forme d'une huile jaune avec un rendement de 65 %. 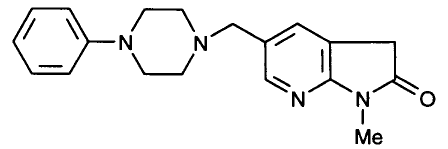
IR (film) : 1719 cm⁻¹ (C=O)
RMN ¹H (CDCl₃), δ (ppm) : 2,57 (t, 4H, 2-CH_{2pipér}, J=5,2 Hz), 3,16 (t, 4H, 2 x CH_{2pipér}, J=5,2 Hz), 3,27 (s, 3H, CH₃), 3,48 (s, 2H, CH₂) ; 3,50 (s, 2H, CH₂), 6,85-6,94 (m, 3H, H_{Ar}), 7,22-7,30 (m, 2H, H_{Ar}) ; 7,52 (s, 1H, H₄) ; 8,06 (s, 1H, H₆).

### EXEMPLE 47 : 1,3-DIHYDRO-5-[2-(4-PHENYLPIPERAZIN-1-YL)ACETYL]-1-METHYL-2H-PYRROLO[2,3-b]PYRIDIN-2-ONE

Ajouter successivement 63 mg (3,9.10⁻⁴ moles) de N-phénylpipérazine puis très lentement 0,08 ml (5,57.10⁻⁴ moles) de triéthylamine à une solution préalablement préparée de 100 mg (3,72.10⁻⁴ moles) de 1,3-dihydro-5-bromoacétyl-1-méthyl-*2H*-pyrrolo[2,3-b] pyridin-2-one (préparation 19) dans 3 ml de dioxane. Après 30 minutes d'agitation à température ambiante, le précipité formé est éliminé par filtration et le filtrat concentré sous pression réduite de manière à obtenir le produit du titre avec un rendement de 78 %. 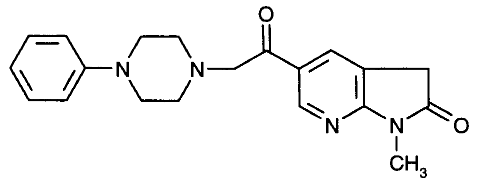
F : 136-137°C
RMN ¹H (CDCl₃), δ (ppm) : 2,77-2,81 (m, H, CH_{2pipér.}) , 3,24-3,28 (m, 4H, CH_{2pipér.}) ; 3,36 (s, 3H, N-CH₃) 3,59 (s, 2H, CH₂-C=O) ; 3,79 (s, 2H, CH₂-N) ; 6,86-6,99 (m, 3H, Hₐᵣₒₘ) ; 7,25-7,34 (m, 2H, Hₐᵣₒₘ) ; 8,12 (s, 1H, H₄) ; 9,05 (s, 1H, H₆)

### EXEMPLE 48 : 1,3-DIHYDRO-5-[2-(4-PHENYLPIPERAZIN-1-YL)ACETYL]-1,3,3-TRIMETHYL-2H-PYRROLO[2,3-b]PYRIDIN-2-ONE

En procédant comme pour l'exemple 47 mais en remplaçant la 1,3-dihydro-5-bromoacétyl-1-méthyl-*2H*-pyrrolo[2,3-b]pyridin-2-one par la 1,3-dihydro-5-bromoacétyl-1,3,3- triméthyl-*2H*-pyrrolo[2,3-b]pyridin-2-one (préparation 23) on obtient le composé du titre. 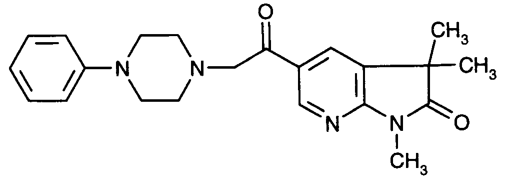
RMN ¹H (CDCl₃), δ (ppm) : 1,45 (s, 6H, 2-CH₃) ; 2,75-2,80 (m, 4H, 2-CH_{2pipér.}) ; 3,25-3,30 (m, 4H, 2-CH_{2pipér.}) ; 3,35 (s, 3H, N-CH₃) ; 3,80 (s, 2H, CH₂-C=O) 6,85-6,95 (m, 3H, Hₐᵣₒₘ) ; 7,25-7,30 (m, 2H, Hₐᵣₒₘ) ; 8,06 (s, 1H, H₄) ; 9,05 (s, 1H, H₆)

### EXEMPLE 49 : 1,3-DIHYDRO-5-[2-(4-PHENYLPIPERAZIN-1-YL)-1-HYDROXYETHYL] -1-METHYL-2H-PYRROLO[2,3-b]PYRIDIN-2-ONE

Ajouter 8 mg (2 10⁻⁴ mole) de borohydrure de sodium à une solution préalablement préparée de 50 mg (1,43 10⁻⁴ moles) 1,3-dihydro-5-[2-(4-phénylpipérazin-1-yl)acétyl]-1-méthyl-*2H*-pyrrolo[2,3-b]pyridin-2-one à 0°C. Après 2 heures d'agitation à température ambiante, ajouter 1 ml d'acide acétique et mettre à sec le milieu réactionnel sous pression réduite. Le produit brut obtenu est purifié par chromatographie sur gel de silice (éluant : méthanol/dichlorométhane, 5/95). Le produit du titre est obtenu sous forme d'huile avec un rendement de 40 %. 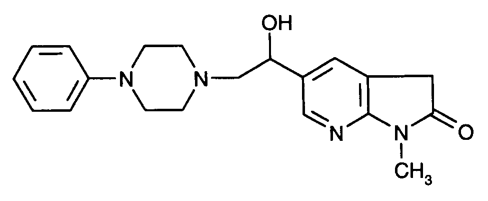
RMN ¹H (CDCl₃), δ (ppm) : 2,57-2,62 (m, 4H, CH_{2pipér.}, CH₂N) , 2,63-2,72 (m, 2H, CH_{2pipér.}) ; 2,91-3,02 (m, 2H, CH_{2pipér.}) 3,24-3,29 (m, 5H, N-CH₃, CH_{2pipér.}) 3,53 (s, 2H, CH₂-C=O) ; 4,79-4,84 (m, 1H, CH-OH), 6,86-6,94 (m, 3H, Hₐᵣₒₘ.) ; 7,25-7,30 (m, 2H, Hₐᵣₒₘ), 7,59 (s, 1H, H₄) ; 8,14 (s, 1H, H₆)

### ETUDE PHARMACOLOGIQUE DES DERIVES DE L'INVENTION

### A) RECHERCHE DE L'ACTIVITE ANALGESIQUE

1) Crampes induites à l'acide acétique
   Le potentiel analgésique de ces produits a été recherché selon le test de KOSTER qui est basé sur le comptage des crampes abdominales induites chez le rat par injection intrapéritonéale d'acide acétique (Koster R., Anderson M., et De Beer E., *J. Fed. Proc.,* (1959), *18*, 412).
   Des rats mâles Wistar randomisés en lot de 5 (poids 150 ± 10 g) reçoivent les produits à tester per os 30 min. avant l'injection intrapéritonéale de 1 cm³ d'acide acétique à 1 %.
   Le nombre de crampes est compté durant les 25 minutes qui suivent l'injection.
   Le pourcentage d'activité a été évalué pour chaque composé (% de diminution du nombre de crampes chez les traités par rapport aux témoins).
2) Crampes induites à la phényl benzoquinone
   Le potentiel analgésique de ces produits a également été recherché selon le test de SIEGMUND qui est basé sur le comptage des crampes induites chez la souris par injection intrapéritonéale de phénylbenzoquinone. (Siegmund E., Cadmus R., *Proc. Sol. Exp. Biol.* *Med.,* (1957), 95, 729).
   Des souris mâles CD-1 randomisées en lot de 5 reçoivent les produits à tester per os 30 min. avec l'injection intrapéritonéale de 0,25 cm³ d'une solution à 0,01 % de phényl benzoquinone dans un mélange eau-éthanol 95-5.
   Le nombre de crampes est compté entre la 5ème et la 15ème minute après l'injection de phénylbenzoquinone.

Le pourcentage d'activité a été évalué pour chaque composé (% de diminution du nombre de crampes chez les traités par rapport aux témoins).

| **PRODUIT** | **DOSE (mg/kg)** | **Acetic Acid Writhing % d'inhibition** | **PBQ Whrithing % d'inhibition** |
|---|---|---|---|
| Aspirine | 50 | 56 % | 62 % |
| Exemple 5 | 50 | 85 % | 85 % |
| Exemple 6 | 50 | 88 % | |
| Exemple 13 | 50 | 96 % | |
| Exemple 16 | 50 | 98 % | 57 % |
| Exemple 20 | 50 | | 100 % |
| Exemple 22 | 50 | 97 % | 53 % |
| Exemple 24 | 50 | 86 % | 97 % |
| Exemple 46 | 50 | | 67 % |
| Exemple 47 | 50 | | 90 % |

Il apparaît que les composés de l'invention possèdent une activité antalgique très intéressante, très significativement supérieure à celle de l'aspirine.

### B) ETUDE DE LA TOXICITE AIGUE

La toxicité aiguë a été appréciée par administration orale de doses croissantes des composés à tester à des lots de 3 souris NMRI mâles.

Les animaux ont été observés à intervalles réguliers durant les 24 heures suivant l'administration de produit.

Il apparaît que les composés de l'invention semble particulièrement atoxiques, aucun décès n'étant observé jusqu'à une dose de 1024 mg/kg avec les composés testés.

### C) COMPRIMES DOSES A 15 mg DE 6-BENZOYL-3-METHYLOXAZOLO[4,5-b] PYRIDIN-2(3H)-ONE

Formule de préparation pour 1000 comprimés :

| | |
|---|---|
| 6-benzoyl-3-méthyloxazolo[4,5-b]pyridin-*2(3H)*-one | 15 g |
| Amidon de blé | 15 g |
| Amidon de maïs | 15 g |
| Lactose | 65 g |
| Stéarate de magnésium | 1 g |
| Silice | 1 g |
| Hydroxy propyl cellulose | 2 g |

### D) COMPRIMES DOSES A 5 mg DE 6-BENZOYL-3-METHYLOXAZOLO[4,5-b] PYRIDIN-2(3H)-ONE

Formule de préparation pour 1000 comprimés :

| | |
|---|---|
| 6-benzoyl-3-méthyloxazolo[4,5-b]pyridin-*2(3H)*-one | 5 g |
| Caféine | 120 g |
| Amidon de maïs | 66 g |
| Lactose | 305 g |
| Stéarate de magnésium | 1 g |
| Silice | 1 g |
| Hydroxy propyl cellulose | 2 g |

## Revendications

1. Composés de formule générale (I) : 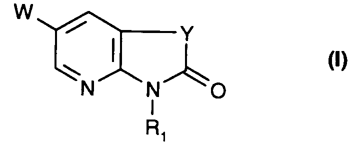 dans laquelle :
- R₁ est choisi parmi l'hydrogène un radical alkyle, alcényle, cyanoalkyle et arylalkyle,
- W est choisi parmi les groupements ―A―R₂ et 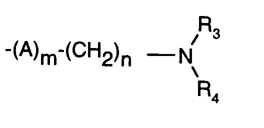
- R₂ est choisi parmi un groupement alkyle, cycloalkyle, cycloalkylalkyle, phényle, phénylalkyle, naphtyle et naphtylalkyle,
- R₃ et R₄ sont choisis indépendamment l'un de l'autre parmi l'hydrogène, un groupement alkyle, phényle, phénylalkyle, cycloalkyle et cycloalkylalkyle ou forment ensemble avec l'atome d'azote qui les porte un système hétérocyclique choisi parmi : 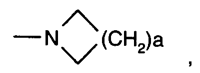 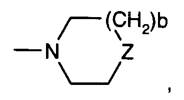 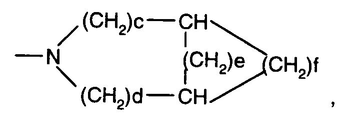 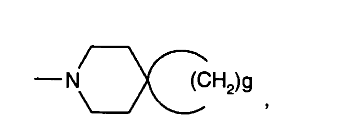 dans lesquels :
- a représente un entier compris entre 0 et 4,
- b représente 1 ou 2,
- c, d, e, f, représentent des nombres entiers compris entre 0 et 4,
- g représente 4 ou 5,
- Z représente O, S ou N-R₇ dans lequel R₇ représente un atome d'hydrogène, un groupement alkyle, phényle, phénylalkyle, cycloalkyle, cycloalkylalkyle, benzhydryle, naphtyle, pyridyle, pyrimidyle,
- n représente un entier de 1 à 4 inclus,
- m représente 0 ou 1,
- A est choisi parmi le groupement 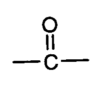 et le groupement 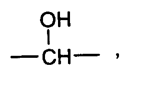
- Y représente un atome d'oxygène ou un groupe 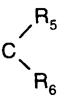 dans lequel R₅ et R₆ sont choisis indépendemment l'un de l'autre parmi l'hydrogène, un radical alkyle, cycloalkyle, cycloalkylalkyle, le radical phényle et le radical benzyle,
étant entendu que lors de la description de la formule (I) :
- les termes "alkyle", "alcényle" et "alkoxy" désignent des groupements linéaires ou ramifiés contenant de 1 à 6 atomes de carbone et pouvant être non substitués ou substitués par un ou plusieurs radicaux alkoxy,
- le terme "aryle" désigne les radicaux phényle, naphtyle ou pyridine,
- les radicaux phényle, benzyle, phénylalkyle, naphtyle, pyridine, pyrimidyle et benzhydryle peuvent être non substitués ou substitués par un ou plusieurs atomes d'halogène ou radicaux hydroxyle, alkyle, alkoxy, trifluorométhyle ou nitro,
- le terme "cycloalkyle" désigne un système cyclique comportant de 3 à 8 atomes de carbone,
- les termes "cycloalkylalkyle", "arylalkyle", "phénylalkyle" et "naphtylalkyle" désignent un cycloalkyle, un aryle, un phényle ou un naphtyle rattaché par l'intermédiaire d'une chaine carbonée linéaire ou ramifiée comportant de 1 à 6 atomes de carbone,
- le système hétérocyclique formé par R₃ et R₄ peut être non substitué ou substitué par un ou plusieurs atomes d'halogène ou radicaux alkyle,
leurs éventuels isomères géométriques et/ou optiques, sous forme pure ou sous forme de mélange, et leurs sels d'addition à un acide ou une base, pharmaceutiquement acceptables.

2. Composés selon la revendication 1 pour lesquels Y représente un atome d'oxygène, leurs éventuels isomères géométriques et/ou optiques sous forme pure ou sous forme de mélange, ainsi que leurs éventuels sels d'addition à un acide ou une base, pharmaceutiquement acceptables.

3. Composés selon la revendication 1 pour lesquels Y représente le groupement 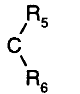 leurs éventuels isomères géométriques et/ou optiques sous forme pure ou sous forme de mélange, ainsi que leurs éventuels sels d'addition à un acide ou une base, pharmaceutiquement acceptables.

4. Composé selon la revendication 1 qui est la 3-méthyl-6-acétyloxazolo[4,5-b]pyridin-*2(3H)*-one.

5. Composé selon la revendication 1 qui est la 6-acétyloxazolo[4,5-b]pyridin-*2(3H)*-one, ainsi que ses sels d'addition à un acide, pharmaceutiquement acceptables.

6. Composé selon la revendication 1 qui est la 3-méthyl-6-benzoyloxazolo[4,5-b]pyridin-*2(3H)*-one.

7. Composé selon la revendication 1 qui est la 6-benzoyloxazolo[4,5-b]pyridin-*2(3H)*-one,
ainsi que ses sels d'addition à un acide, pharmaceutiquement acceptables.

8. Composé selon la revendication 1 qui est la 5-benzoyl-1-méthyl-1,3-dihydro-*2H*-pyrrolo[2,3-b]pyridin-2-one.

9. Composé selon la revendication 1 qui est la 3-méthyl-6-[(4-phénylpipérazin-1-yl)méthyl]oxazolo[4,5-b]pyridin-*2(3H)*-one,
ainsi que ses sels d'addition à un acide, pharmaceutiquement acceptables.

10. Composé selon la revendication 1 qui est la 3-méthyl-6-[3-(4-phénylpipérazin-1-yl) prop-1-yl]oxazolo[4,5-b]pyridin-*2(3H)*-one,
ainsi que ses sels d'addition à un acide, pharmaceutiquement acceptables.

11. Composé selon la revendication 1 qui est la 3-méthyl-6-[2-(4-phénylpipérazin-1-yl)acétyl]oxazolo[4,5-b]pyridin-*2(3H)*-one,
ainsi que ses sels d'addition à un acide, pharmaceutiquement acceptables.

12. Composé selon la revendication 1 qui est la 3-méthyl-6-[2-(4-phénylpipérazin-1-yl)1-hydroxyéthyl]oxazolo[4,5-b]pyridin-*2(3H)*-one, ainsi que ses sels d'addition à un acide, pharmaceutiquement acceptables.

13. Composé selon la revendication 1 qui est la 1,3-dihydro-1-méthyl-5-(4-phénylpipérazin-1-ylméthyl)-*2H*-pyrrolo[2,3-b]pyridin-2-one,
ainsi que ses sels d'addition à un acide, pharmaceutiquement acceptables.

14. Composé selon la revendication 1 qui est la 1,3-dihydro-1-méthyl-5-[2-(4-phénylpipérazin-1-yl)-1-hydroxy-éthyl]-*2H*-pyrrolo[2,3-b]pyridin-2-one,
ainsi que ses sels d'addition à un acide, pharmaceutiquement acceptables.

15. Procédé de préparation des composés de formule (I) selon la revendication 1 caractérisé en ce que l'on fait réagir :
***A. lorsque W représente -A-R₂,*** un composé de formule (II) : 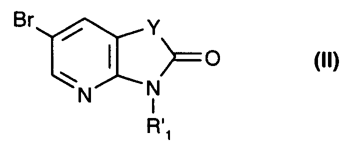 dans laquelle Y est tel que défini dans la formule (I) et R'₁ a la même définition que R₁ avec la réserve que R'₁ ne peut représenter un atome d'hydrogène,
• soit en présence de 1,2-bis(diphénylphosphino)éthane et d'acétate de palladium (II) avec un éther insaturé de formule (III) :
Alk―O―CH=CH―R'₂ **(III)**
dans laquelle Alk représente un alkyle de 1 à 4 atomes de carbone et R'₂ représente un hydrogène ou un alkyle linéaire ou ramifié de 1 à 5 atomes de carbone éventuellement substitué par un phényle, naphtyle ou cycloalkyle, de manière à obtenir les composés de formule (IV) : 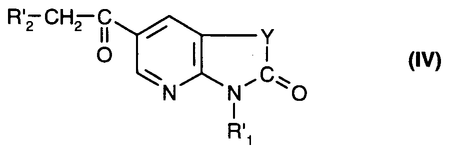 dans laquelle R'₁, R'₂ et Y sont tels que définis précédemment,
• soit en présence de tétrakis(triphénylphosphine)palladium et de chlorure de lithium avec un composé de formule (V) : 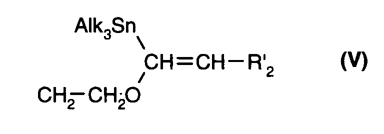 dans laquelle R'₂ et Alk ont la même définition que précédemment de manière à accéder également aux composés de formule (IV) : 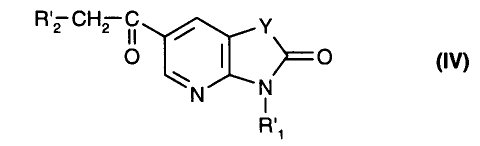
• soit en présence de zinc avec un dérivé halogéné de formule (VI) :
Ar-CH₂―Hal **(VI)**
dans laquelle Hal représente un atome d'halogène et Ar un groupement phényle ou naphtyle éventuellement substitué de manière à obtenir les composés de formule (VII) : 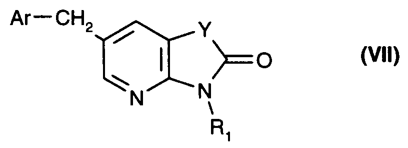 dans laquelle Ar est tel que défini précédemment et R₁ est tel que défini dans la formule (I),
que l'on soumet à une réaction d'oxydation avec un agent oxydant pour accéder aux composés de formule (VIII) : 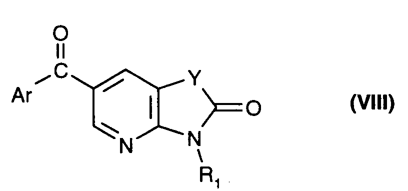 dans laquelle Ar, Y et R₁ sont tels que défini précédemment,
avec dans le cas où le N-bromosuccinimide est utilisé et où Y représente le groupement CH₂, nécessité de faire suivre la réaction d'oxydation d'une étape de débromation avec du zinc,
les composés de formules (IV) et (VIII) pouvant si on le désire :
- dans le cas où R₁ ou R'₁ représentent un groupement benzyle, être débenzylés en présence de palladium sur charbon et hydrogène pour accéder aux composés de formules (IX) et (X) : 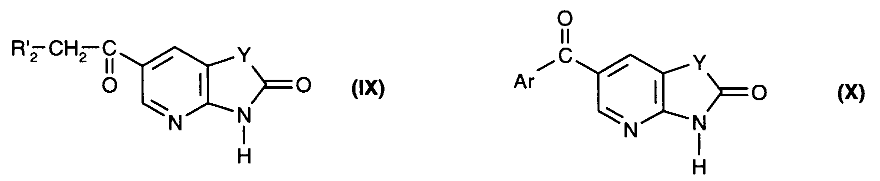 dans lesquelles Y, Ar et R'₂ sont tels que définis précédemment,
- dans le cas où R₁ ou R'₁ représentent un groupement cyanométhylé, être décyanométhylé en présence d'oxyde de platine et d'hydrogène pour accéder à ces mêmes composés de formules (IX) et (X),
l'ensemble de ces composés (IV), (VIII), (IX), (X) constituant les composés de formule (XI) : 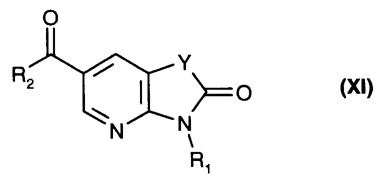 dans laquelle R₁, R₂ et Y sont tels que définis précédemment,
composés de formule (XI) qui peuvent, si on le désire, être réduits par un agent réducteur, en alcool de formule (XII) : 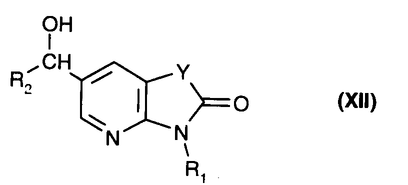 dans laquelle R₁, R₂ et Y sont tels que définis précédemment,
***B. lorsque W représente*** 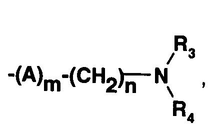 un composé de formule (XIII) : 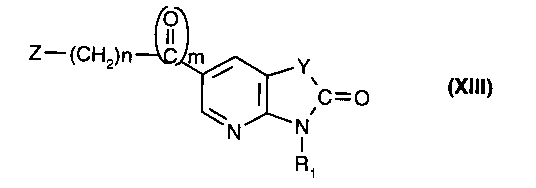 dans laquelle Y, R₁, n et m sont tels que définis pour la formule (I) et Z représente un groupement partant tel qu'un atome d'halogène ou un tosylate, avec une amine de formule (XIV) : 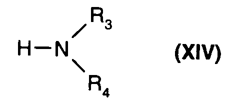 dans laquelle R₃ et R₄ sont tels que définis pour la formule (I),
pour obtenir un composé de formule (XV) : 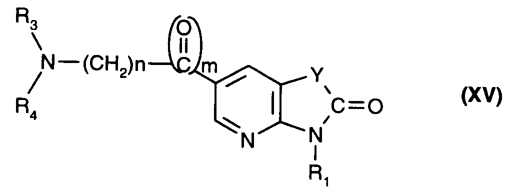 dans laquelle n, m, Y, R₁, R₃ et R₄ sont tels que défini précédemment,
dont on peut éventuellement, dans le cas où m = 1, réduire la fonction carbonyle avec un agent réducteur, pour obtenir l'alcool correspondant de formule (XVI) : 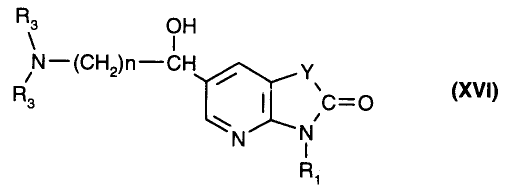 dans laquelle n, Y, R₁, R₂ et R₃ sont tels que définis précédemment,
l'ensemble des composés de formules (XI), (XII), (XV) et (XVI) formant les composés de formule (I) que l'on purifie le cas échéant par une technique classique de purification, dont on sépare, si on le souhaite, les isomères géométriques et les isomères optiques par une technique classique de séparation et que l'on transforme, si nécessaire, en leurs sels d'addition à un acide ou à une base, pharmaceutiquement acceptables.

16. Composition pharmaceutique contenant comme principe actif au moins un des composés selon la revendication 1, sous forme de base ou salifié, et en combinaison avec un ou plusieurs excipients ou véhicules inertes non toxiques pharmaceutiquement acceptables.

17. Composition pharmaceutique selon la revendication 16, caractérisé en ce que le principe actif est associé à de la caféine en combinaison avec un ou plusieurs excipients ou véhicules inertes non toxiques pharmaceutiquement acceptables.

18. Composition pharmaceutique selon les revendications 16 et 17 utile pour le traitement d'algies.
